# EUROPEAN PATENT SPECIFICATION

(11) **EP 0 597 506 B1**
(45) Date of publication and mention of the grant of the patent: **16.12.1998**
(21) Application number: 93119270.2
(22) Date of filing: 18.10.1989
(51) Int. Cl.: A61M 29/02

(54) **Catheter equipped with expansible member and method of manufacturing the same**
Katheter mit ausdehnbarem Element und dessen Herstellungsverfahren
Cathéter muni d'un élément expansible et procédé pour sa fabrication

(30) Priority: 20.10.1988 JP 264888/88; 22.05.1989 JP 129827/89; 02.08.1989 JP 200995/89
(43) Date of publication of application: 18.05.1994
(62) Divisional of application: 89119330.2
(73) Proprietor: TERUMO KABUSHIKI KAISHA, Tokyo 151 (JP)
(72) Inventor: Sugiyama, Yoshiaki, Fujinomiya-shi, Shizuoka-ken (JP); Sagae, Kyuta, Fuji-Shi, Shizuoka-ken (JP)
(74) Representative: Casalonga, Axel

(56) References cited:
- EP-A- 0 266 957
- WO-A-88/06465
- US-A- 4 597 755
- US-A- 4 639 252

## Description

This invention relates to a catheter equipped with an expansible member for use in curing a stricture portion inside a blood vessel by expanding the stricture portion to improve the blood flow on the peripheral side of the stricture portion, and to a method of manufacturing the same.

Heretofore, as a catheter equipped with an expansible member for expanding a stricture portion inside a blood vessel, so-called Gruentich type disclosed, for example, in U.S. Patent Specification No.4,195,637, or so-called Simpson-Robert type disclosed, for example, in U.S. Patent Specification No.4,323,071 has been used. However, adaptable cases of vasodilatation using such a catheter were hitherto limited to those of localized stricture near coronary artery from an anatomical viewpoint, which were lesions of about 15 to 20 mm length, monobranched lesions, non-calcified lesions, etc. Catheters of the above-mentioned types have been improved in order to extend the range of the adaptable cases. For this purpose, a catheter so-called low-profile shape which has the same structure as those of standard structures of the above-mentioned types but only the tip portion of which is narrowed, was developed. The catheter of this low-profile shape is adaptable to strictures in more peripheral blood vessel or more severe strictures (sub-complete clogging).

In addition, the applicant of this application has proposed a catheter comprising an inner tube, an outer tube disposed coaxially with the inner tube, an expansible member attached to the inner and outer tubes, and a rigidity imparting member consisting of braided metal wire and disposed on the inner or outer tube as disclosed in the International Publication No.WO 88/06465.

In a catheter called Gruentich type as described above, an expansible member is attached to the tip portion of a catheter tube having two lumens. One of the lumens is open at the tip thereof to form a passage for a guide wire and for tip pressure measurement. The other of the lumens is in communication with the expansible member at the base end portion of the expansible member to form a passage for fluid such as vasographic contrast liquid injected under pressure for expanding the expansible member. The catheter of this type is made of flexible synthetic resin.

A catheter called Simpson-Robert type has a coaxial double-wall structure comprising an inner tube having a first lumen whose tip is open, and an outer tube which forms a second lumen between it and the inner tube and to the tip of which an expansible member is attached. An ultrafine metal pipe is disposed in the second lumen for removing bubbles therefrom. The catheter of this type is also made of flexible synthetic resin like the above-mentioned Gruentich type catheter.

As described above, a catheter to be inserted into a blood vessel is generally made of flexible synthetic resin for facilitating the insertion and avoiding an injury to the wall of the blood vessel. However, such a catheter has a possibility of being flexed in a blood vessel upon insertion because of its flexibility. Further, there is a case that a delicate movement of the tip of the catheter is required for making it reach the aimed portion in the blood vessel. For this purpose, the catheter must be moved or rotated at its base end portion in a delicate manner and the force or torque for the movement or rotation must be transmitted from the base end portion to the tip portion of the catheter. In a conventional catheter, however, the transmission of the force or torque is bad because the force or torque is apt to be absorbed owing to the flexibility of the catheter. Therefore, the conventional catheter is disadvantageous in its delicate operability.

The catheter disclosed in the above-mentioned International Publication No.WO 88/06465 solves those problems of the conventional Gruentich or Simpson-Robert type catheter by disposing the rigidity imparting member. In this catheter, the flexion or collapse of the catheter is suppressed and the transmission of the force or torque is improved. However, the flexibility of this catheter is decreased by the rigidity imparted by the rigidity imparting member. The decrease of the flexibility lowers the followability of the catheter to curved portion of blood vessel and the operability of the insertion. Further, even this catheter including the rigidity imparting member of braided metal wire has a possibility of meandering in a blood vessel. In that case, the force applied to the base end portion of the catheter for pushing the catheter into the blood vessel is absorbed at the meandering portion. As the result, the operation of the catheter, particularly for advancing the tip portion of the catheter to a stricture portion inside a blood vessel becomes difficult.

Further, the above-mentioned catheter has a substantially equal outer diameter from the base end portion to the tip portion. In general, the physical properties of the catheter of this type are selected in order to meet the requirements for operating the catheter at its base end or intermediate portion. For this reason, in the above-mentioned catheter, the outer diameter at the tip portion is as large as that at the base end portion. As a result, there is a risk that the tip portion of the catheter could not be inserted into a more severe stricture portion of a blood vessel.

The tip portion of the catheter disclosed in the above-mentioned International Publication No.WO 88/06465 is beveled by grinding or with a solvent in order to avoid an injury to the inner surface of a blood vessel upon insertion into a curved portion of the blood vessel. In general, it is preferable that the expansible member of the catheter is disposed as near to the tip of the catheter as possible. For this purpose, it is preferable that the mounting portion between the expansible member and the inner tube is as short as possible. Contrarily, a length to some extent of the mounting portion is required for reliable adhesion between the expansible member and the inner tube. However, beveling the tip portion of the catheter as described above decreases the length for the mounting portion and results in deteriorating the adhesion between the expansible member and the inner tube. As a result, there is a possibility that the necessary expansion of a blood vessel is not attained because of leak of fluid for expansion such as vasographic contrast liquid out of the expansible member at a peeling portion of the expansible member off the inner tube upon operating the catheter. Further, in the above-mentioned catheter, the tip portion of the expansible member at which the expansible member is attached to the inner tube is exposed outside of the catheter. Therefore, there is a possibility that the tip portion of the expansible member partially peels off the inner tube upon insertion into a blood vessel and leads to an injury to the inner surface of the blood vessel.

EP-A-266 957 discloses a balloon dilatation catheter adapted for use in angiography techniques having two balloons at the distal end thereof, one inside the other. The distal extension tube of this catheter is reinforced internally by a helical spring which extends along and within the distal extension tube . The spring reinforces the tube to resist collapse of the tube when the balloons are inflated under high pressures.

Accordingly, it is an object of the present invention to provide a catheter equipped with an expansible member in which the flexibility of the catheter, particularly the elastic deformability in the lateral direction with respect to the axial direction of the catheter is not decreased, an extreme flexion or bend of the catheter is prevented, the force applied to the base end portion of the catheter for advancing the tip portion thereof to a stricture portion inside a blood vessel is surely transmitted to the tip portion, and the operability of the catheter is good.

According to an aspect of the present invention and as claimed a catheter equipped with an expansible member comprises an inner tube having a base end portion and a first lumen those tip is open; an outer tube capable of receiving said inner tube therein, having a base end portion and the tip thereof at a position recessed by a predetermined distance from the tip of said inner tube, and forming a second lumen between it and the outer surface of said inner tube; a foldable expansible member having a tip end portion and a base end portion, said tip end portion of said expansible member being attached to said inner tube, said base end portion of said expansible member being attached to said outer tube, said expansible member communicating with said second lumen at a portion near said base end portion of said expansible member; a first opening formed in said base end portion of said inner tube to communicate with said first lumen; a second opening formed in said base end portion of said outer tube to communicate with said second lumen; and a rigidity imparting member extending in the axial direction. According to the invention said rigidity imparting member consists of a linear member disposed in said second lumen, the tip end portion of the rigidity imparting member being fixed to the tip end portion of the inner tube, the base end portion of the rigidity imparting member being fixed to the inner or outer tube and the intermediate portion between the tip end and the base end portions of said rigidity imparting member being fixed neither to the inner nor to the outer tube and being of linear shape.

According to another aspect of the present invention and as claimed a catheter equipped with an expansible member comprises a catheter tube having a tip end portion, a base end portion, a first lumen the tip of which is open, a second lumen open at a position recessed by a predetermined distance from said tip of said first lumen; a foldable expansible member having a tip end portion and a base end portion, said tip end portion of said expansible member being attached to said tip end portion of said catheter tube, said base end portion of said expansible member being attached to a portion near an opening portion formed near the tip of said catheter tube, said exapnsible member communicating with said second lumen; a first opening formed in said base end portion of said catheter tube to communicate with said first lumen; a second opening formed in said base end portion of said catheter tube to communicate with said second lumen; and a rigidity imparting member extending in the axial direction. According to the invention said rigidity imparting member consists of a linear member disposed in at least one of said first and second lumens, and the tip end portion of the rigidity imparting member is fixed to the tip end portion of said catheter tube, the base end portion of the rigidity imparting member is fixed to the base end portion of said catheter tube, and the intermediate portion between the tip end and the base end portions of said rigidity imparting member is not fixed to the catheter tube and is of linear shape.

The above and other objects, features and advantages of the present invention will be better understood from the following description taken in conjunction with the accompanying drawings, in which:
Fig. 1 is an enlarged cross sectional view of the tip end portion of an embodiment of a catheter equipped with an expansible member according to the present invention;
Fig. 2 is a view of the base end portion of the catheter of Fig. 1;
Fig. 3 is a cross sectional view of the intermediate portion of the catheter of Fig. 1;
Fig. 4 is a cross sectional view taken along line I-I of Fig. 1;
Fig. 5 is a cross sectional view taken along line II-II of Fig. 1;
Fig. 6 is a cross sectional view of one example of the base end portion of the catheter of the present invention;
Fig. 7 is a side elevational view of one example of a rigidity imparting member used in the catheter of the present invention;
Fig.8 is an enlarged cross sectional view taken along line X-X of Fig.7;
Fig.9 is an enlarged cross sectional view taken along line XI-XI of Fig.7;
Figs.10, 11 and 12 are side elevational views of other examples of rigidity imparting members used in catheters of the present invention;
Fig.13 is a cross sectional view of another embodiment of a catheter equipped with an expansible member according to the present invention;
Fig.14 is an enlarged cross sectional view of the tip end portion of another embodiment of a catheter equipped with an expansible member according to the present invention;
Fig.15 is a cross sectional view taken along line III-III of Fig.14;
Fig.16 is a cross sectional view taken along line IV-IV of Fig.14;
Fig.17 is a cross sectional view of the base end portion of the catheter of Fig. 14;
Figs.18 through 21 are views for illustrating a step of forming the rear outer tube of the catheter of Fig.3;
Figs.22 through 25 are views for illustrating a step of forming the front outer tube of the catheter of Fig.3;
Figs.26 through 30 are views for illustrating a step of connecting the rear outer tube to the front outer tube of the catheter of Fig.3;
Figs.31 through 34 are views for illustrating a step of forming the expansible member of the catheter of Fig.1;
Fig.35 is a view for illustrating a step of attaching the expansible member to the outer tube of the catheter of Fig.1;
Fig.36 is a view for illustrating a step of attaching the expansible member to the inner tube of the catheter of Fig.1;
Figs. 37 through 41 are views for illustrating the operation of the catheter of Fig. 1.

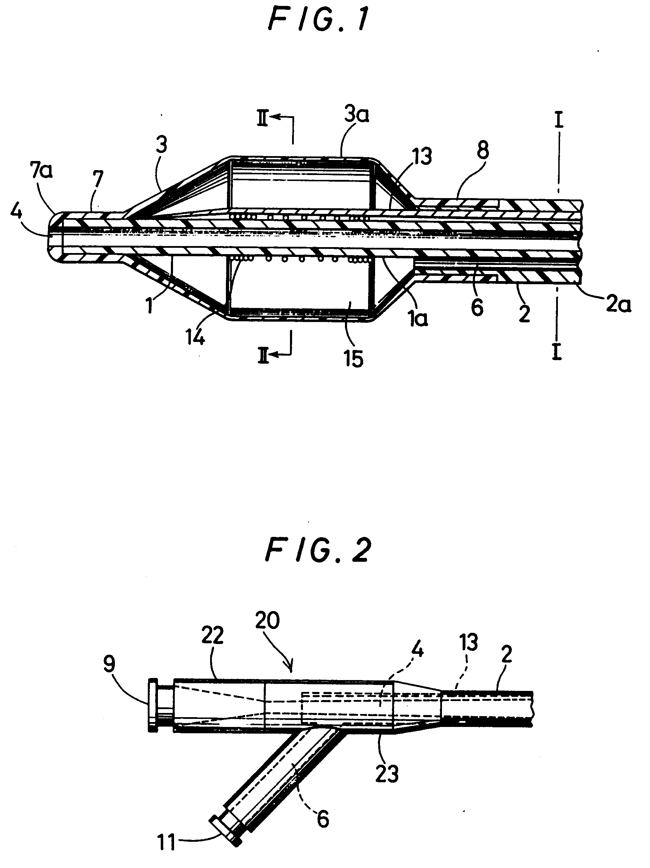

The present invention will be described as to preferred embodiments shown in the attached drawings and particularly Figs. 1 to 6.

A catheter equipped with an expansible member according to the embodiments comprises an inner tube 1 having a base end portion and a first lumen 4 whose tip is open, an outer tube 2 capable of receiving the inner tube 1 therein, having a base end portion and the tip thereof at a position recessed by a predetermined distance from the tip of the inner tube 1, and forming a second lumen 6 between it and the outer surface of the inner tube 1, a foldable expansible member 3 having a tip end portion 7 and a base end portion 8, the tip end portion 7 of the expansible member 3 being attached to the inner tube 1, the base end portion 8 of the expansible member 3 being attached to the outer tube 2, the expansible member 3 communicating with the second lumen 6 at a portion near the base end portion 8 of the expansible member 3, a first opening 9 formed in the base end portion of the inner tube 1 to communicate with the first lumen 4, a second opening 11 formed in the base end portion of the outer tube 2 to communicate with the second lumen 6, and a rigidity imparting member 13 consisting of a linear member extending in the axial direction disposed in the second lumen 6.

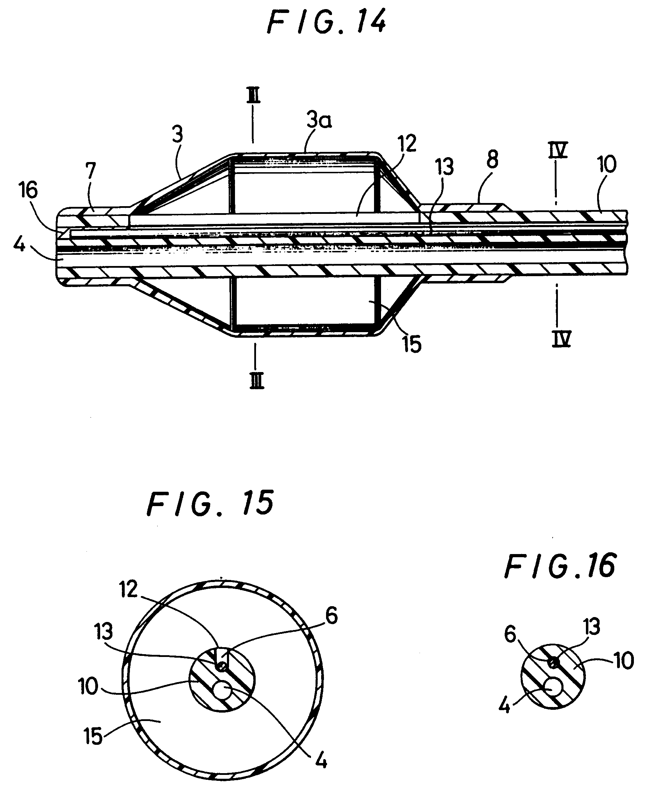

Further, a catheter equipped with an expansible member according to the embodiments as shown in Figs. 14 to 17 comprises a catheter main body having a tip end portion and a base end portion and comprising an inner tube 1 having a tip end portion, a base end portion and a first lumen 4 whose tip is open, an outer tube 2 capable of receiving the inner tube 1 therein, having a tip end portion, a base end portion and the tip thereof at a position recessed by a predetermined distance from the tip of the inner tube 1, and forming a second lumen 6 between it and the outer surface of the inner tube 1, and a foldable expansible member 3 having a tip end portion 7 and a base end portion 8, the tip end portion 7 of the expansible member 3 being attached to the inner tube 1, the base end portion 8 of the expansible member 3 being attached to the outer tube 2, the expansible member 3 communicating with the second lumen 6 at a portion near the base end portion 8 of the expansible member 3, a first opening 9 formed in the base end portion of the inner tube 1 to communicate with the first lumen 4, and a second opening 11 formed in the base end portion of the outer tube 2 to communicate with the second lumen 6, the outer diameter of the tip end portion of the catheter main body including a portion to which the expansible member 3 is attached is smaller than that of the base end portion of the catheter main body.

A catheter equipped with an expansible member according to one embodiment of the present invention comprises a catheter main body including an inner tube 1, an outer tube 2 and an expansible member 3, and a branched hub 20.

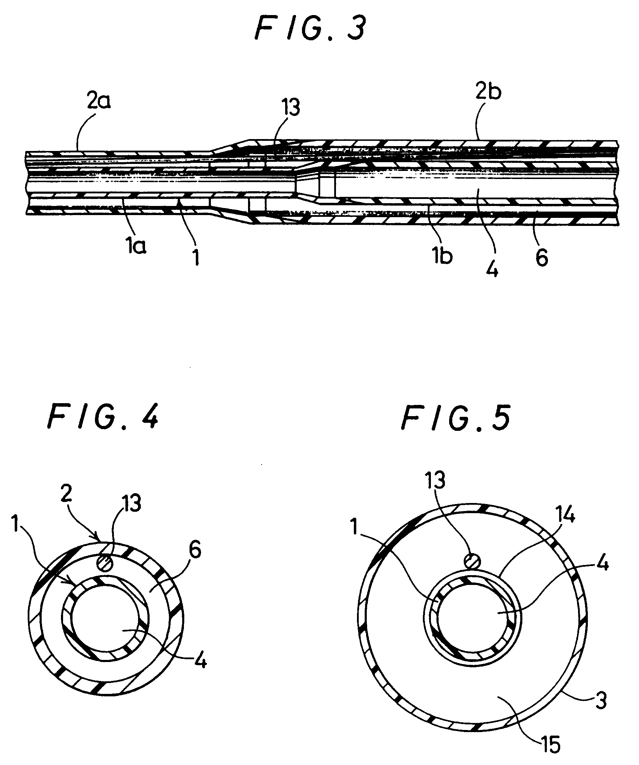

The inner tube 1 has a first lumen 4 whose tip is open. The first lumen 4 is a lumen for inserting a guide wire therein and in communication with a first opening 9 which forms a guide wire port disposed in the branched hub 20. It is preferable that the outer diameter of the tip end portion of the inner tube 1 is smaller than that of the base end portion thereof. In this embodiment, as shown in Fig.3, the inner tube 1 consists of a front inner tube 1a and a rear inner tube 1b. The outer tube 2 described later also consists of a front outer tube 2a and a rear outer tube 2b. The outer diameters of the front inner and outer tubes 1a and 2a are smaller than those of the rear inner and outer tubes 1b and 2b respectively. Therefore, the catheter of this embodiment can be inserted into more peripheral blood vessel than conventional one. It is preferable that the tip end portion of the catheter having the small outer diameter is as long as the distance from the inlet of a coronary artery to an aimed lesion part, particularly, a little longer than the distance from the inlet of the coronary artery to the aimed lesion part. Specifically, the length of the tip end portion is preferably about 50 to 700 mm, more preferably 80 to 400 mm, more preferably 100 to 300 mm. In addition, it is preferable that each of the inner and outer tubes is provided with a tapering portion at the connecting portion between the tip end portion 1a or 2a and the base end portion 1b or 2b to make the change of its inner and outer diameters smooth.

The front inner tube 1a of the inner tube 1 has preferably an outer diameter of 0.30 to 2.00 mm, more preferably 0.40 to 1.80 mm, and an inner diameter of 0.20 to 1.80 mm, more preferably 0.25 to 1.60 mm. The rear inner tube 1b has preferably an outer diameter of 0.40 to 2.50 mm, more preferably 0.55 to 2.40 mm, and an inner diameter of 0.25 to 2.35 mm, more preferably 0.30 to 1.80 mm.

As the inner tube, a tube may be used which is made into one body by extrusion molding such that the outer diameter of the tip end portion of the tube is smaller than that of the base end portion thereof, instead of the tube 1 consisting of the front and rear inner tubes 1a and 1b. Also in that case, for making the change of the outer diameter of the tube smooth, it is preferable that a tapering portion is formed by the extrusion molding at the portion at which the outer diameter changes.

The material for forming the inner tube 1 preferably has a certain extent of flexibility. Thermoplastic resins can be used, for example, polyolefin such as polyethylene, polypropylene, ethylene-propylene copolymer and ethylene-vinyl acetate copolymer, polyvinyl chloride, polyamide elastomer and polyurethane; or silicone rubber, latex rubber, etc. The above-mentioned thermoplastic resin is preferable and the above-mentioned polyolefin is more preferable.

The outer tube 2 into which the inner tube 1 is inserted has a tip slightly recessed by a predetermined distance from the tip of the inner tube 1. As shown in Fig.4 which is a cross section taken along line I-I in Fig. 1, a second lumen 6 is formed by the inner surface of the outer tube 2 and the outer surface of the inner tube 1. Thus, the second lumen 6 has a sufficient volume. The tip portion of the second lumen 6 is in communication with the rear end portion of the inside of the expansible member 3 described later. The rear end portion of the second lumen 6 is in communication with a second opening 11 provided to the branched hub 20 to form an injection port for injecting fluid for expanding the expansible member (for example, vasographic contrast liquid), As shown in Fig.3, the outer tube 2 consists of a front outer tube 2a and a rear outer tube 2b. The outer diameter of the front outer tube 2a is smaller than that of the rear outer tube 2b so as to facilitate insertion of the tip end portion of the catheter into more peripheral blood vessel. The front outer tube 2a of the outer tube 2 has preferably an outer diameter of 0. 50 to 4.00 mm, more preferably 0.60 to 3.70 mm, and an inner diameter of 0.40 to 3. 50 mm, more preferably 0.50 to 2.70 mm. The rear outer tube 2b has preferably the outer diameter of 0.75 to 4.30 mm, more preferably 1.00 to 4.00 mm, and the inner diameter of 0.70 to 3.80 mm, more preferably 0.80 to 3.00 mm. Also as the outer tube, a tube may be used which is made into one body by extrusion molding such that the outer diameter of the tip end portion of the tube is smaller than that of the base end portion thereof, instead of the tube 1 consisting of the front and rear outer tubes 2a and 2b. In that case, for making the change of the outer diameter of the tube smooth, it is preferable that a tapering portion is formed by the extrusion molding at the portion at which the outer diameter changes.

The material for forming the outer tube 2 preferably has a certain extent of flexibility. Thermoplastic resins can be used, for example, polyolefin such as polyethylene, polypropylene, ethylene-propylene copolymer and ethylene-vinyl acetete copolymer, polyvinyl chloride, polyamide elastomer and polyurethane; or silicone rubber, latex rubber, etc. The above-mentioned thermoplastic resin is preferable and the above-mentioned polyolefin is more preferable.

As shown in Figs.1 and 3 through 6, a rigidity imparting member 13 is provided in the second lumen 6 formed by the inner surface of the outer tube 2 and the outer surface of the inner tube 1. The rigidity imparting member 13 extends from the base end portion to the tip end portion of the catheter. The rigidity imparting member 13 is for preventing an extreme flexion and meandering of the catheter main body in a blood vessel without considerable decrease of the flexibility of the catheter and for facilitating insertion of the tip end portion of the catheter into a stricture portion inside a blood vessel.

As shown in Figs.1 and 3, the rigidity imparting member 13 consists of a linear member. The linear member preferably consists of metal wire, for example, made of elastic metal such as stainless steel, super elastic alloy, etc. desirably of the wire diameter of 0.05 to 1.50 mm, more desirably of 0.10 to 1.00 mm. Particulaly, it is preferably made of high tensile spring stainless steel, Cu or NI-Ti alloy. The tip end portion of the rigidity member 13 is fixed to prevent the tip end portion of the rigidity imparting member 13 from damaging the expansible member 3 and so that the force applied to the base end portion of the catheter for advancing the tip end portion of the catheter is surely transmitted to the tip end portion of the catheter. In the embodiment shown in Fig.1, the tip end portion of the rigidity imparting member 13 is slenderer than the other portion thereof by, for example, grinding. The slenderer portion of the rigidity imparting member 13 is located between the tip end portion 7 of the expansible member 3 and the inner tube 1, and fixed to the tip end portion of the inner tube 1 together with the expansible member 3. The slenderer portion of the rigidity imparting member 13 prevents a step-like change of the outer surface of the expansible member 3 in its mounting portion. The average diameter of the slenderer portion is preferably about 1/5 to 1/10 of the diameter of its neighboring portion. It is preferable that the rigidity of the major body portion of the rigidity imparting member 13 is higher than that of the tip end portion thereof. This purpose can be attained by, for example, using a rigidity imparting member in which the cross section of its major body portion is larger than that of its tip end portion. Otherwise, to obtain a rigidity imparting member in which its tip end portion is relatively flexible and its major body portion has a high rigidity, a rigidity imparting member may be used in which the metal wire for the rigidity imparting member has been annealed with a temperature gradient so that the temperature for the tip end portion is high and the temperature for the major body portion is low after cold working.

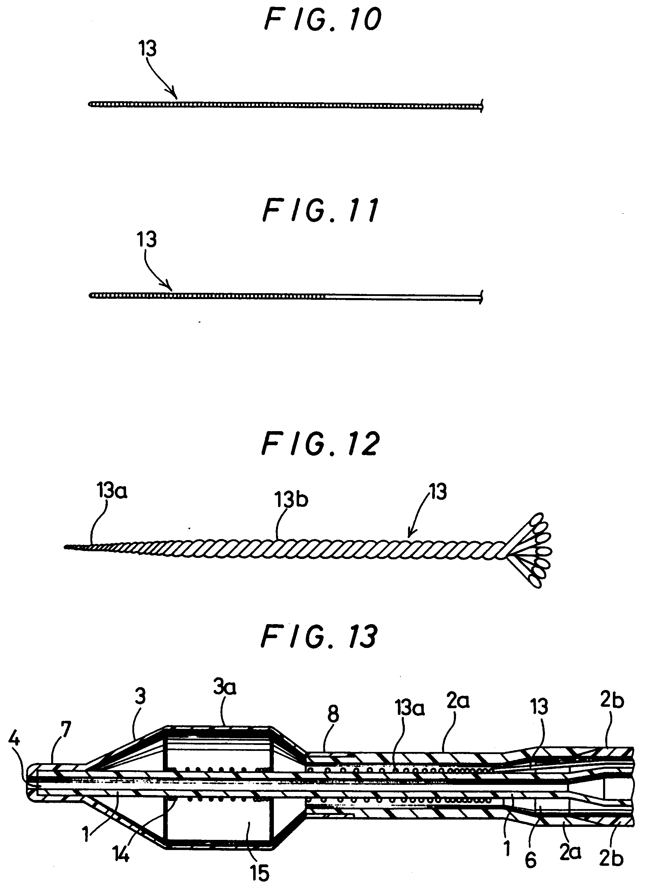

As the rigidity imparting member 13, a strand wire consisting of several fine metal wires braided may be used as shown in Fig.12. In that case, for making the tip end portion 13a of the rigidity imparting member 13 flexible and the major body portion 13b have a high rigidity, it is preferable that the diameter of the tip end portion 13a is smaller than that of the major body portion 13b. For this purpose, metal wires to be braided the diameter of the tip end portion of each of which is smaller than that of the major body portion may be used, or the number of metal wires braided may be decreased toward the tip end portion.

It is preferable that the portion except the tip and base end portions of the rigidity imparting member 13 is not fixed. By not fixing this intermediate portion of the rigidity imparting member 13, when the tip end portion of the catheter is bent, the rigidity imparting member 13 does not hamper the flexibility of the tip end portion of the catheter because the rigidity imparting member 13 can slip within the second lumen.

Such a rigidity imparting member 13 prevents the catheter main body from meandering in a blood vessel. Therefore, because the force applied to the base end portion of the catheter main body is not absorbed at the meandering portion, the force can be surely transmitted to the tip end portion of the catheter. As a result, the operability of the catheter, particularly the operation of advancing the tip end portion of the catheter to which the expansible member is attached, to a stricture portion inside a blood vessel becomes easy. In addition, insertion of the tip end portion of the catheter into a more severe stricture portion (subcomplete clogging) becomes possible.

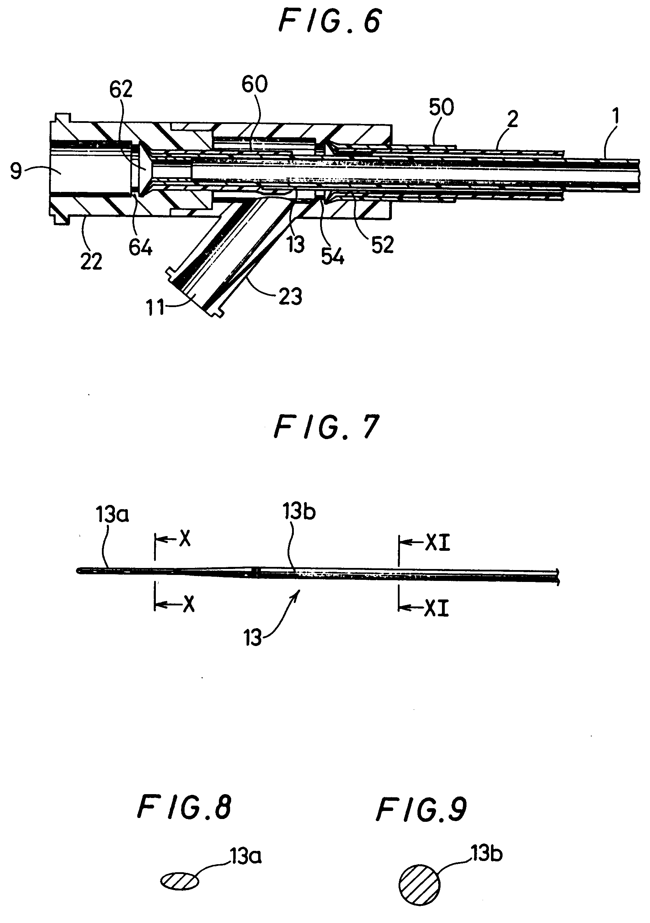

The rigidity imparting member 13 is not limited to that of the embodiment shown in Fig.1. For example, a rigidity imparting member 13 shown in Fig.7 is also usable adequately. In the rigidity imparting member 13 shown in Fig.7, the tip end portion is slenderer than the other portion of the rigidity imparting member 13 like that shown in Fig.1. In this example, however, the slenderer portion 13a is an oblate slenderer portion 13a having an elliptic cross section as shown in Fig.8 which is a cross sectional view taken along line X-X of Fig.7. By forming the tip end portion of the rigidity imparting member 13 into the oblate slenderer portion 13a, mounting it between the inner tube 1 and the tip end portion of the expansible member 3 becomes easy. The major body portion 13b of the rigidity imparting member 13 has a substantially circular cross section as shown in Fig.9 which is a cross sectional view taken along line XI-XI of Fig.7. Further, the rigidity imparting member 13 may be a coil spring type the whole of which consists of a fine metal wire as shown in Fig.10. In addition, as shown in Fig.11, a rigidity imparting member 13 may be used only the tip end portion of which consists of a fine metal wire into a coil spring and the major body portion of which consists of a linear metal wire.

The tip end portion of the rigidity imparting member 13 may not be fixed between the inner tube 1 and the tip end portion of the expansible member 3 as described above. For example, as shown in Fig. 13, the tip end portion 13a of the rigidity imparting member 13 may be fixed by winding it around the outer surface of the inner tube 1. In that case, it is preferable that the tip end portion 13a of the rigidity imparting member 13 is fixed by winding it around a portion near the portion of the inner tube 1 corresponding to the portion of the outer tube 2 the diameter of which is decreased, and the tip end portion 13a of the rigidity imparting member 13 is wound to the portion of the inner tube 1 corresponding to the base end portion of the expansible member 3. By winding the tip end portion 13a of the rigidity imparting member 13 around the outer surface of the portion of the inner tube 1 the outer diameter of which is decreased, a flexion of the slenderer portion of the inner tube 1 is prevented so that a flexion of the slenderer portion of the outer tube 2 is prevented. In order to make the tip side portion of the slenderer portion of the inner tube 1 flexible, as shown in Fig.13, it is preferable to wind the tip end portion 13a of the rigidity imparting member 13 around the outer surface of the inner tube 1 such that it becomes close gradually from the tip end portion to the base end portion of the inner tube 1.

The expansible member 3 is foldable and it is folded on the outer circumference of the inner tube 1 in its non-expanded state. The expansible member 3 has a substantially cylindrical portion 3a having an approximatelly uniform diameter at least a part of which is substantially cylindrical for enabling to expand a stricture portion in a blood vessel. The substantially cylindrical portion 3a described above may not be completely cylindrical but may be polygonal. The base end portion 8 of the expansible member 3 is secured in a liquid-tight manner to the tip end portion of the outer tube 2 by adhesion, fusion or the like. The tip end portion 7 of the expansible member 3 is also secured in a liquid-tight manner to the tip end portion of the inner tube 1. As shown in Fig.5 which is a cross sectional view taken along line II-II of Fig.1, the expansible member 3 forms an expanded space 15 between the inner surface of the expansible member 3 and the outer surface of the inner tube 1. The expanded space 15 is in communication over the entire circumference at its rear end portion with the second lumen 6. Thus, the rear end of the expansible member 3 is in communication with the second lumen 6 having a relatively large volume. Therefore, it is easy to inject expansion fluid through the second lumen 6 into the expansible member 3. The material for forming the expansible member 3 preferably has a certain extent of flexibility. The materials which can be used are thermoplastic resins, for example, polyolefin such as polyethylene, polypropylene, ethylene-propylene copolymer and ethylene-vinyl acetate copolymer, polyvinyl chloride, cross-linked ethylene-vinyl acetate copolymer, polyamide elastomer and polyurethane; or silicone rubber, latex rubber, etc. The above-mentioned thermoplastic resin is preferable and the above-mentioned cross-linked ethylene-vinyl acetate copolymer is more preferable. It is preferable that the forward and backward portions of the cylindrical portion 3a of the expansible member 3 are tapered. As the size of the expansible member 3, the cylindrical portion upon expanded has an outer diameter of 1.00 to 35.00 mm, preferably 1.50 to 30.00 mm and a length of 3.00 to 80.00 mm, preferably 10.00 to 75.00 mm. The entire length of the expansible member 3 is 5.00 to 120.00 mm, preferably 15.00 to 100.00 mm.

It is preferable that a marker 14 is provided on the outer surface of the inner tube 1. As shown in Fig. 1, the marker 14 is disposed from a portion near the portion on the rear side of the mounting portion between the expansible member 3 and the inner tube 1 to a portion near the portion on the tip end side of the mounting portion between the expansible member 3 and the outer tube 2. That is, the marker 14 has a length as long as that of the cylindrical portion 3a of the expansible member 3. The marker 14 is made of X-ray impermeable material (for example, gold, platinum, tungsten or alloy of them, or silver-palladium alloy). The marker 14 preferably consists of a coil spring. In that case, it is more preferable that the coil spring is wound closely in ranges of 1 to 4 mm, more preferably 2 to 3 mm from both ends of marker 14. This is for enabling to confirm easily the position of the expansible member 3 under X-ray perspection. Further, by forming the marker 14 into a spring shape, it functions as a reinforcement for preventing the inner tube within the expansible member from flexing or collapsing at its bend portion. Particularly, when the marker 14 consists of a spring coil wound around the inner tube 1 closely, the resistance against an external force becomes higher. Further, when the cross section of the coiled wire is circular, rectangular or elliptic, the resistance against an external force becomes much higher.

As shown in Fig.2, the branched hub 20 comprises an inner tube hub 22 secured to the inner tube 1 and having a first opening 9 which is in communication with the first lumen 4 to form a guide wire port, and an outer tube hub 23 secured to the outer tube 2 and having a second opening 11 which is in communication with the second lumen 6 to form an injection port. The inner and outer tube hubs 22 and 23 are connected to each other. For the material for forming the branched hub, there can be suitably used thermoplastic resin such as polycarbonate, polyamide, polysulfone, polyarylate and methacrylate-butylene-styrene copolymer. A cross section of an embodiment of the branched hub 20 is shown in Fig. 6. In this embodiment, a flexion-preventing tube 50 is disposed on one end portion of the outer tube 2. The flexion-preventing tube 50 is made of shrinkable material such that the inner diameter thereof after shrinkage is a little smaller than the outer diameter of the outer tube 2. The flexion-preventing tube 50 can be easily attached by the manner that the shrinkable tube 50 is put on one end portion of the outer tube 2 and then shrinked by heating, for example, with blowing hot air. The outer tube 2 to which the flexion-preventing tube 50 has been attached is fixed to the outer tube hub 23 with a lock member 52, which has an enlarged rear end portion. The outer diameter of the portion except the rear end portion of the lock member 52 is as large as the inner diameter of the outer tube 2. For fixing the outer tube 2, after the lock member 52 is inserted in the rear end portion of the outer tube 2, the outer tube 2 is inserted from its tip end in the outer tube hub 23 till the enlarged rear end portion of the lock member 52 passes on a projection 54 formed on the inner surface of the outer tube hub 23. The outer tube 2 may be bonded to the outer tube hub 23 with an adhesive applied to the outer surface of the flexion-preventing tube 50. For the material for forming the outer tube hub 23, there can be suitably used thermoplastic resin such as polycarbonate, polyamide, polysulfone, polyarylate and methacrylate-butylene-styrene copolymer.

A flexion-preventing tube 60 is disposed on one end portion of the inner tube 1. The tube 60 is made of shrinkable material such that the inner diameter thereof after shrinkage is a little smaller than the outer diameter of the inner tube 1. The flexion-preventing tube 60 can be easily attached by the manner that the shrinkable tube 60 is put on one end portion of the inner tube 1 and then shrinked by heating, for example, with blowing hot air. The base end portion of the rigidity imparting member 13 is secured to the outer surface of the inner tube 1 with the flexion-preventing tube 60. The base end portion of the rigidity imparting member 13 may not be secured. The inner tube 1 to which the flexion-preventing tube 60 has been attached is fixed to the inner tube hub 22 with a lock member 62, which has an enlarged rear end portion. The outer diameter of the portion except the rear end portion of the lock member 62 is as large as the inner diameter of the inner tube 1. For fixing the inner tube 1, after the lock member 62 is inserted in the rear end portion of the inner tube 1, the inner tube 1 is inserted from its tip end in the inner tube hub 22 till the enlarged rear end portion of the lock member 62 passes on a projection 64 formed on the inner surface of the inner tube hub 22. The inner tube 1 may be bonded to the inner tube hub 22 with an adhesive applied to the outer surface of the flexion-preventing tube 60. For the material for forming the inner tube hub 22, there can be suitably used thermoplastic resin such as polycarbonate, polyamide, polysulfone, polyarylate and methacrylate-butylene-styrene copolymer. As shown in Fig.6, the inner and outer tube hubs 22 and 23 are connected to each other by the manner that the inner tube hub 22 is inserted from its tip end in the rear end portion of the outer tube hub 23 attached to the base end portion of the outer tube 2. In this case, an adhesive may be previously applied to the connecting portion between the inner and outer tube hubs 22 and 23 to make a reliable adhesion between them. Instead of the branched hub 20, for example, tubes each of which comprises a port member having an opening on its rear end may be connected in liquid-tight manner to the first and second lumens, respectively.

Next, an embodiment of a catheter equipped with an expansible member shown in Figs.14 through 17 will be described.

A catheter equipped with an expansible member according to this embodiment comprises a catheter tube 10 having a tip end portion, a base end portion, a first lumen 4 the tip of which is open, a second lumen 6 open at a position recessed by a predetermined distance from the tip of the first lumen 4; a foldable expansible member 3 having a tip end portion 7 and a base end portion 8, the tip end portion 7 of the expansible member 3 being attached to the tip end portion of the catheter tube 10, the base end portion 8 of the expansible member 3 being attached to a portion near an opening portion 12 formed near the tip of the catheter tube 10, the expansible member 3 communicating with the second lumen 6; a first opening formed in the base end portion of the catheter tube 10 to communicate with the first lumen 4; a second opening 11 formed in the base end portion of the catheter tube 10 to communicate with the second lumen 6; and a rigidity imparting member 13 consisting of a linear member extending in the axial direction disposed in at least one of the first and second lumens.

Further, a catheter equipped with an expansible member according to this embodiment comprises a catheter tube 10 having a tip end portion, a base end portion, a first lumen 4 at least the tip of which is open, a second lumen 6 open at a position recessed by a predetermined distance from the tip of the first lumen 4; a foldable expansible member 3 having a tip end portion 7 and a base end portion 8, the tip end portion 7 of the expansible member 3 being attached to the tip end portion of the catheter tube 10, the base end portion of the expansible member 3 being attached to a portion near an opening portion 12 formed near the tip of the catheter tube 10, the expansible member 3 communicating with the second lumen 6; a first opening 9 formed in the base end portion of the catheter tube 10 to communicate with the first lumen 4; and a second opening 11 formed in the base end portion of the catheter tube 10 to communicate with the second lumen 6, the outer diameter of the tip end portion of the catheter tube 10 including at least a portion to which the expansible member 3 is attached is smaller than that of the base end portion of the catheter tube 10.

A catheter equipped with am expansible member according to this embodiment comprises a catheter main body including a catheter tube 10 and an expansible member 3, and a branched hub 20.

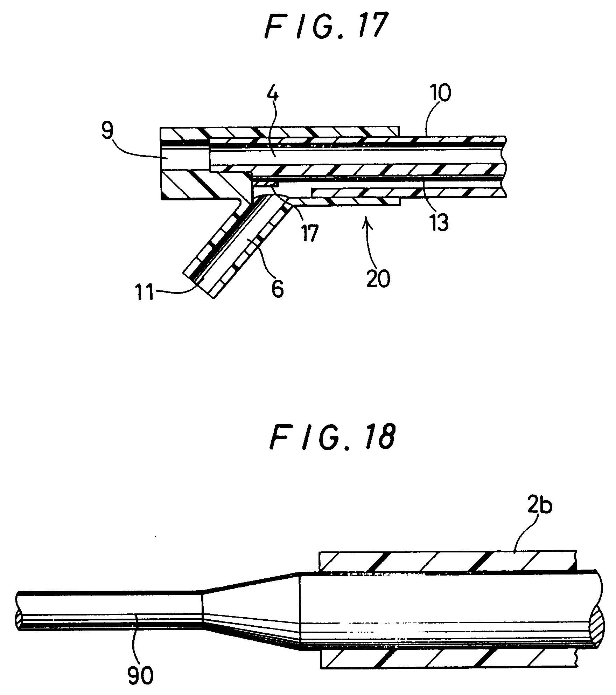

The catheter tube 10 has a first lumen 4 whose tip is open and a second lumen 6 which is in communication with the inside of the expansible member 3 through an opening portion 12 at a position somewhat recessed from the tip to the base end side. As shown in Fig.16 which is a cross sectional view taken along line IV-IV of Fig.14, the first and second lumens 4 and 6 are formed within the catheter tube 10. The first lumen 4 is a lumen for inserting a guide wire therein and in communication with a first opening 9 which forms a guide wire port disposed in the branched hub 20. The tip portion of the second lumen 6 is in communication with the rear end portion of the inside of the expansible member 3. As shown in Fig.17, the rear end portion of the second lumen 6 is in communication with a second opening 11 provided to the branched hub 20 to form an injection port for injecting fluid for expanding the expansible member (for example, vasographic contrast liquid). It is preferable that the outer diameter of the tip end portion of the catheter tube 10 is smaller than that of the base end portion thereof. It is more preferable that a tapering portion is formed between the tip and base end portions of the catheter tube 10 to make the change of its inner and outer diameters smooth. The tip end portion of the catheter tube 10 has preferably the outer diameter of 0.5 to 4.0 mm, more preferably 0.6 to 3.7 mm. The inner diameter of the first lumen is preferably 0.2 to 2.25 mm, more preferably 0.25 to 1.6 mm. The inner diameter of the second lumen is preferably 0.1 to 1.45 mm, more preferably 0.3 to 1.3 mm. The base end portion of the catheter tube 10 has preferably an outer diameter of 0.75 to 4.3 mm, more preferably 1.0 to 4.0 mm. The inner diameter of the first lumen is preferably 0.1 to 2.35 mm, more preferably 0.3 to 1.8 mm. The inner diameter of the second lumen is preferably 0.1 to 1.7 mm, more preferably 0.3 to 1.6 mm. The catheter tube 10 is formed by, for example, extrusion molding. The material for forming the catheter tube 10 preferably has a certain extent of flexibility. Thermoplastic resins can be used, for example, polyolefin such as polyethylene, polypropylene, ethylene-propylene copolymer and ethylene-vinyl acetate copolymer, polyvinyl chloride and polyurethane; or polyamide elastomer, silicone rubber, latex rubber, etc. The above-mentioned thermoplastic resin is preferable and the above-mentioned polyolefin is more preferable.

As shown in Figs.14 through 17, a rigidity imparting member 13 is provided in the second lumen 6. The rigidity imparting member 13 is for preventing an extreme flexion or meandering of the catheter main body in a blood vessel without considerable decrease of the flexibility of the catheter and for facilitating insertion of the tip end portion of the catheter into a stricture portion inside a blood vessel. Although the rigidity imparting member 13 is preferably disposed in the second lumen 6, it may be disposed in the first lumen 4. In any case, the rigidity imparting member 13 described before can be suitably used.

The tip end portion 7 of the expansible member 3 is secured to the catheter tube 10 on the front side of the opening 12 while the base end portion 8 thereof is secured to the catheter tube 10 on the base end side of the opening 12. The opening 12 extends in the axial direction of the catheter tube 10 by a predetermined length. Also in this embodiment, the expansible member 3 described before can be suitably used. As shown in Fig.15 which is a cross sectional view taken along line III-III of Fig.14, the expansible member 3 forms an expanded space 15 between the inner surface of the expansible member 3 and the outer surface of the inner tube 1. The tip end portion of the second lumen 6 of the catheter tube 10 is filled with a filler 16 to be closed. The tip end portion of the rigidity imparting member 13 is located in the filler 16 and fixed. The tip end portion of the rigidity imparting member 13 is fixed so that the tip end portion of the rigidity imparting member 13 is prevented from being am injury to the expansible member 3 and the transmission of the force, which is applied to the base end portion of the catheter for advancing the catheter, to the tip end portion is improved.

The branched hub 20 as shown in Fig.17 is attached to the rear end portion of the catheter tube 10. The rear end portion of the rigidity imparting member 13 disposed in the second lumen 6 of the branched hub 20 is fixed with a filler 17. The rear end portion of the rigidity imparting member 13 may not be fixed. For the material for forming the branched hub, there can be suitably used thermoplastic resin such as polycarbonate, polyamide, polysulfone, polyarylate and methacrylate-butylene-styrene copolymer.

Next, a method of manufacturing a catheter equipped with an expansible member of the present invention will be described with reference to the drawings.

A method of manufacturing a catheter equipped with an expansible member according to this embodiment comprises a step of forming an inner tube having a tip end portion and a lumen extending from the tip end to the base end of said inner tube; a step of forming an outer tube having a tip end portion, a lumen extending from the tip end to the base end of said outer tube, an inner diameter larger than the outer diameter of said inner tube, and a length smaller than that of said inner tube by a predetermined length; a step of forming a contractible or foldable expansible member having a tip end portion and a base end portion; a step of inserting said inner tube in said outer tube; a step of attaching said base end portion of said expansible member to said tip end portion of said outer tube; and a step of attaching said tip end portion of said expansible member to said tip end portion of said inner tube, said step of forming said outer tube comprising a step of toning a front outer tube having a lumen extending from the tip end to the base end thereof, a step of forming a rear outer tube having an outer diameter larger than that of said front outer tube and a lumen extending the tip end to the base end of said rear outer tube, a step of tapering one end of said rear outer tube, a step of enlarging the diameter at one end of said front outer tube, and a step of connecting the tapered end of said rear outer tube to the enlarged end of said front outer tube.

Each step will be described with the catheter shown in Fig.1.

First, the step of forming an inner tube 1 having a lumen 4 extending from the tip end to the base end of the inner tube 1 can be taken place by a method of cutting a tube member ride of thermoplastic resin for the inner tube 1 by extrusion molding into a predetermined length or by an injection molding method.

The step of toning an outer tube 2 comprises a step of forming a front outer tube 2a having a lumen extending from the tip end to the base end of the tube 2a; a step of forming a rear outer tube 2b having an outer diameter larger than that of the front outer tube 2a and a lumen extending from the tip end to the base end of the tube 2b; a step of tapering one end of the rear outer tube 2b; a step of enlarging the diameter of one end portion of the front outer tube 2a; and a step of connecting the tapering end of the rear outer tube 2b to the enlarged end of the front outer tube 2a.

Each of the front and rear outer tubes 2a and 2b can be formed by a method of cutting a tube member made of thermoplastic resin for the outer tube 2 by extrusion molding into a predetermined length or by an injection molding method. In the step of tapering the tip end portion of the rear outer tube 2b, as shown in Fig.18, a tapering core 90 is inserted in the rear outer tube 2b. The tapering core 90 is used for preventing a formation of a step or the like between the front and rear outer tubes 2a and 2b upon fitting the tapering portion 92 of the rear outer tube 2b to a cone-shaped enlarged portion 94 of the front outer tube 2a as described later, and making the diameter change smooth. The tapering core 90 may be made of glass or metal. As shown in Fig.18, the tapering core 90 is inserted till one end of the rear outer tube 2b reachs a position near a tapering portion of the tapering core 90.

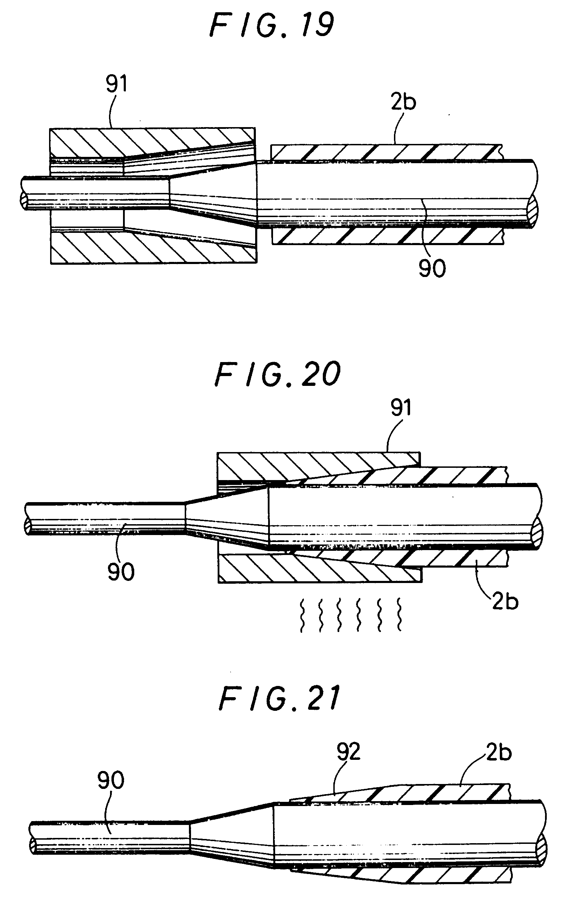

For tapering the tip end portion of the rear outer tube 2b, a tapering mold 91 as shown in Fig.19 is used. The tapering mold 91 may be made of glass or metal. It is preferable that the tapering mold 91 has a tapering inner surface corresponding to the tapering portion of the tapering core 90 for making the diameter change of the rear outer tube 2b smooth.

As shown in Fig.20, the tapering mold 91 is made to press the tip end portion of the rear outer tube 2b and heated by a heater such as a heat gun and a hot air torch (not shown) to taper the tip end portion of the rear outer tube 2b. Fig.21 shows the tip end portion of the rear outer tube 2b after removing the tapering mold 91, wherein the tip end portion of the rear outer tube 2b has a tapering portion 92.

Next, the step of enlarging the rear end portion of the front outer tube 2a into a cone shape will be described.

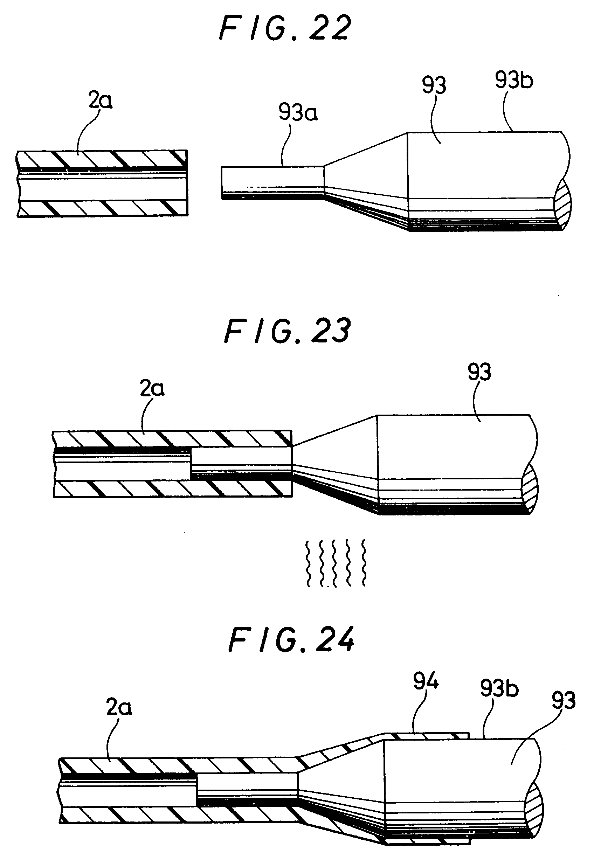

In this step, as shown in Fig.22, a slender portion 93a of an enlarging pin 93 is inserted in one end of the front outer tube 2a. As shown in Pig.22, the enlarging pin 93 comprising the slender portion 93a, a tapering portion and a thick portion 93b. The diameter of the slender portion 93a is as large as or a little smaller than the inner diameter of the front outer tube 2a. The diameter of the thick portion 93b is as large as or a little larger than the outer diameter of the rear outer tube 2b. The enlarging pin 93 is inserted from the slender portion 93a in one end of the front outer tube 2a gradually, and, as shown in Fig.23, the one end portion of the front outer tube 2a and the tapering portion of the enlarging pin 93 is heated by a heater such as a heat gun and a hot air torch (not shown) to enlarge the diameter of the one end portion of the front outer tube 2a. Particularly, in the case of the front outer tube 2a having a large thickness, it is preferable that the enlarging pin 93 is pushed in with heating otherwise the front outer tube 2a may be split along the axial direction.

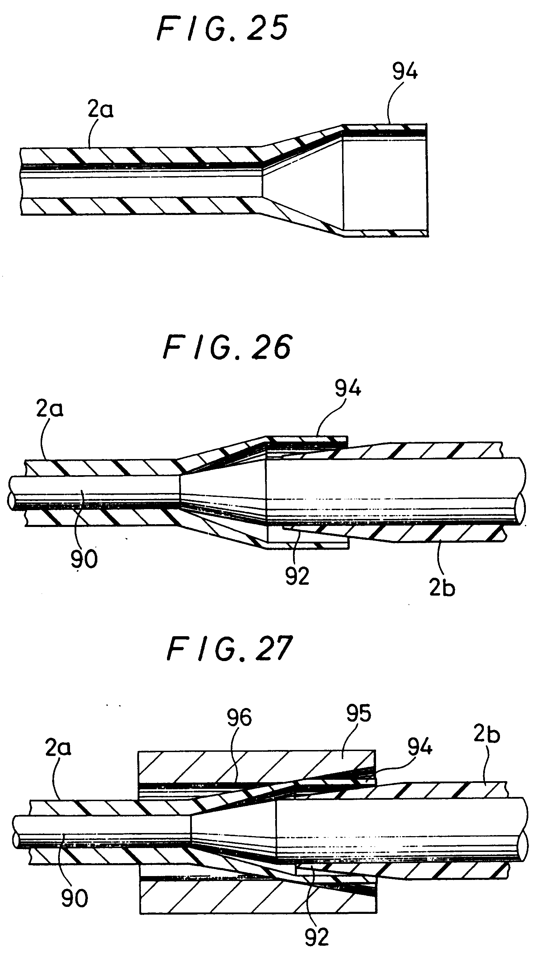

The above operation is continued till the thick portion 93b of the enlarging pin 93 is inserted in the one end portion of the front outer tube 2a. The length of the one end portion of the front outer tube 2a overlapping the thick portion 93b of the enlarging pin 93 is preferably as long as or a little smaller than the length of the tapering portion 92 formed in the rear outer tube 2b. This makes possible to prevent a formation of a step on the connecting portion between the cone-shaped enlarging portion 94 of the front outer tube 2a and the tapering portion 92 of the rear outer tube 2b, and make it smooth, as described later. As shown in Fig.25, the front outer tube 2a having the enlarged end portion is obtained by removing the enlarging pin 93.

Next, the step of connecting the tapering portion 92 of the rear outer tube 2b to the enlarged portion 94 of the front outer tube 2a will be described. Fig.26 illustrates this step.

First, the tapering portion 92 of the rear outer tube 2b shown in Fig.21 is inserted together with the tapering core 90 in the enlarged portion 94 of the front outer tube 2a. The outer diameter of the portion of the tapering core 90 inserted in the front outer tube 2a, that is, the outer diameter of the portion of the front side of the tapering portion of the tapering core 90 is as large as or a little smaller than the inner diameter of the front outer tube 2a. This is for obtaining a smooth finish from the front outer tube 2a to the connecting portion, further to the rear outer tube 2b. The leading portion of the tapering portion 92 of the rear outer tube 2b is preferably inserted into the leading portion of the cone-shaped enlarged portion 94 of the front outer tube 2a. It is preferable that the tip end portion of the leading portion of the cone-shaped enlarged portion 94 of the front outer tube 2a is as large as or a little smaller than the length of the tapering portion 92 of the rear outer tube 2b because a smooth finish of the outer tube is obtained and a formation of a step is prevented.

Next, a method for connecting the tapering portion 92 of the rear outer tube 2b to the enlarged portion 94 of the front outer tube 2a will be described.

As an embodiment of this step, a method using a mold for connecting as shown in Fig. 27 will be described.

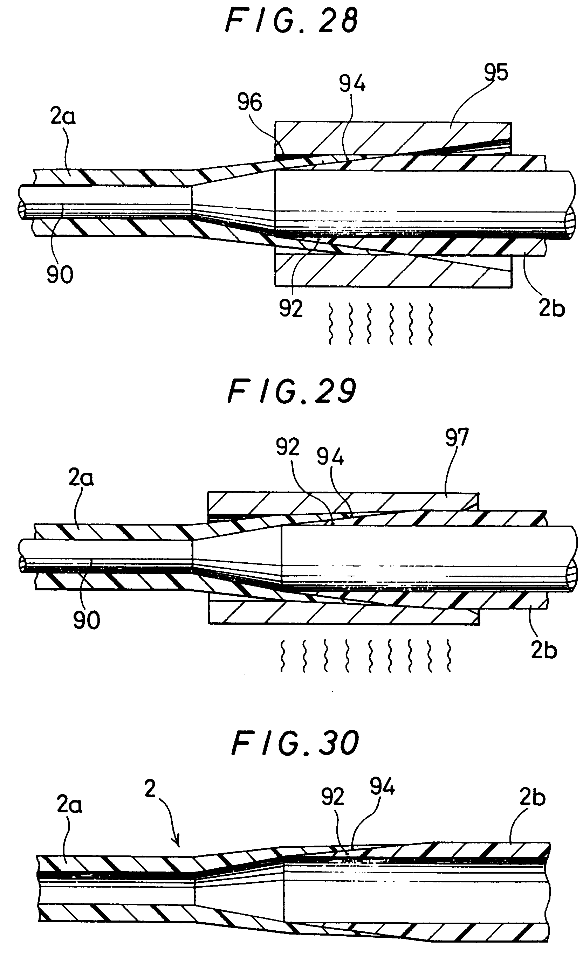

A first connecting mold 95 has a pipe-shape profile. The first connecting mold 95 has an opening at one end wider than an opening at the other end as shown in Fig.27. The inner diameter of the first connecting mold 95 gradually decreases in a cone shape from the one end to an intermediate portion of the first connecting mold 95. An equal inner diameter portion 96 is formed from the intermediate portion to the other end of the first connecting mold 95. The cone-shaped portion of the first connecting mold 95 is for making possible to press gradually the cone-shaped enlarged portion 94 of the front outer tube 2a onto the tapering portion 92 of the rear outer tube 2b. The connecting portion of the front and rear outer tube 2a and 2b, which projects from the outer surface of the rear outer tube 2b because the thickness of the connecting portion becomes larger than the thickness of the rear outer tube 2b, can be easily inserted into the equal inner diameter portion 96 of the first connecting mold 95 because of the cone-shaped tapering portion of the first connecting mold 95. The first connecting mold 95 is for bonding the front and rear outer tube 2a and 2b at the connecting portion with heating. The first connecting mold 95 is preferably made of material capable of being heated from the external, for example, made of glass or metal. The inner diameter of the equal inner diameter portion 96 of the first connecting mold 95 is as large as or a little larger than the outer diameter of the rear outer tube 2b. This is for pressing the projecting portion of the connecting portion of the front and rear outer tubes 2a and 2b. As shown in Fig.28, the equal inner diameter portion 96 of the first connecting mold 95 is put on the connecting portion of the front and rear outer tubes 2a and 2b so that the cone-shaped enlarged portion 94 of the front outer tube 2a is welded to the tapering portion 92 of the rear outer tube 2b by being heated by a heater. Because the outer tube 2 is formed by inserting the tip end portion of the rear outer tube 2b in the rear end portion of the front outer tube 2a and bonding the former to the latter as described above, the connecting portion between the front and rear outer tubes 2a and 2b is hard to come off upon insertion of the catheter.

In the step described above, if a step is formed on the connecting portion of the front and rear outer tubes 2a and 2b, it is preferable to remove the step. For this purpose, a second connecting mold 97 as shown in Fig.29 is preferably used for surface treatment of the connecting portion in the similar manner to that of the first connecting mold 95.

The second connecting mold 97 has a similar shape to that of the first connecting mold 95 but the inner diameter of the second connecting mold 97 is a little smaller than that of the equal inner diameter portion 96 of the first connecting mold 95. This is for pressing strongly the connecting portion of the front and rear outer tubes 2a and 2b to bond them strongly to each other by welding with heat. The outer tube 2 consisting of a tube having different outer diameters as shown in Fig.30 is obtained by removing the connecting mold and the tapering core 90.

In the embodiment shown in Fig.1, the inner tube 1 also consists of a tube having different outer diameters. This inner tube 1 is preferably formed by the similar manner to that for the outer tube 2 described above. The order of the steps of forming the inner and outer tubes is optional. They may be taken place at a time.

Next, the step of forming a contractible or foldable expansible member having a tip end portion and a base end portion will be described.

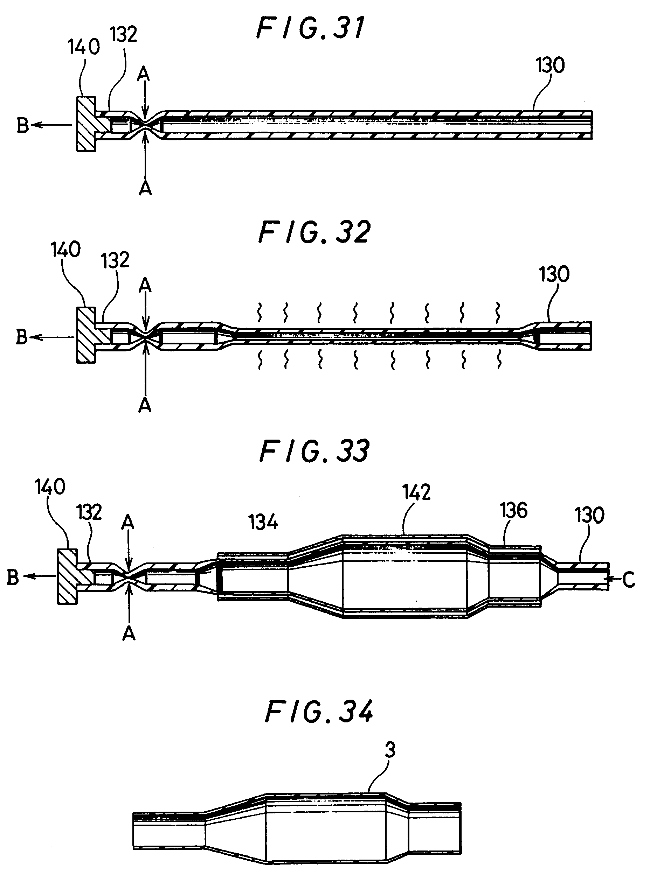

The expansible member 3 preferably has a certain extent of flexibility. For this purpose, the expansible member 3 is preferably made of thermoplastic resin, for example, polyolefin such as polyethylene, polypropylene, ethylene-propylene copolymer, ethylene-vinyl acetete copolymer and cross-linked ethylene-vinyl acetete copolymer; polyvinyl chloride and polyurethane. Particularly, it is preferable that the expansible member 3 is made of cross-linked ethylene-vinyl acetete copolymer. The expansible member 3 can be formed, for example, by the manner shown in Figs. 31 through 34.

First, as shown in Fig.31, a thermoplastic tube 130 for forming the expansible member 3 is formed. A tube holder 140 is attached to one end portion 132 of the tube 130. The lumen of the tube 130 is closed at a portion shown by arrows A near the tube holder 140. The closure is made by, for example, welding with heat, sealing with high frequency, or using a forceps. The tube 130 closed at the portion shown by arrows A is stretched by applying a load to the tube holder 140 to remove slacks from the tube 30. Fig.31 shows the tube 130 from which slacks have been removed. As shown in Fig.32, a portion of the tube 130 which is to form the expansible member 3 is heated to a temperature near the melting point of the material of the tube 130 by a heater (not shown). While keeping the tube 130 heated, as shown in Fig.33, a mold 142 which has an internal shape corresponding to a shape of the expanded expansible member 3 is put on the tube 130 and the heated, portion of the tube 130 is pressed onto the inner surface of the mold 142 by introducing gas under pressure from the direction shown by an arrow C. The tube 130 is kept pressurized till the tube 130 comes back to the room temperature. The mold 142 is removed after the portion of the tube 130 which is to form the expansible member 3 is constricted by making the inside of the tube 130 at a negative pressure. The tube 130 is cut at the tip and rear end portions 134 and 136 to obtain the expansible member 3 as shown in Fig.34.

When at least the tip and base end portions of the expansible member 3 are shrinkable, the expansible member 3 can be easily attached to the inner and outer tubes 1 and 2 by shrinking. For making the tip and base end portions of the expansible member 3 shrinkable, the expansible member 3 may be made of cross-linked thermoplastic resin. Otherwise, in the step of forming the expansible member 3 described above, the portion of the tube 130 which is to form the expansible member 3 may be heated to such a temperature that strains will remain after cooling. By this manner, the whole of the expansible member 3 can be made shrinkable. Further, for making the tip and base end portions of the expansible member 3 shrinkable, after the expansible member is formed such that the inner diameters of the tip and base end portions of the expansible member are a little smaller than outer diameters of the inner and outer tubes 1 and 2, respectively, the both end portions of the expansible member may be enlarged (by drawing). The order of the steps of forming the expansible member and the inner and outer tubes described before is optional.

Next, a step of forming the opening 11 which is in communication with the lumen 6 of the outer tube 2, in the base end portion of the outer tube 2 will be described. It is preferable to form the opening 11 by attaching the outer tube hub 23 having am opening to the base end portion of the outer tube 2. This case will be described with reference to Fig.6.

First, the flexion-preventing tube 50 is attached to one end portion of the outer tube 2. The flexion-preventing tube 50 consists of such a shrinkable tube that the inner diameter thereof after shrinkage is a little smaller than the outer diameter of the outer tube 2. The flexion-preventing tube 50 is attached by the manner that the shrinkable tube 50 is put on the one end portion of the outer tube 2 and then shrinked by heating, for example, with blowing hot air. The outer tube hub 23 is attached to the outer tube 2 to which the flection-preventing tube 50 has been attached. This attachment of the outer tube hub 23 is taken place by using a lock member 52 which has an enlarged rear end portion. The outer diameter of the portion except the rear end portion of the lock member 52 is as large as the inner diameter of the outer tube 2. For attaching the outer tube hub 23 to the outer tube 2, after the lock member 52 is inserted in the rear end portion of the outer tube 2, the outer tube 2 is inserted from its tip end in the outer tube hub 23 till the enlarged rear end portion of the lock member 52 passes on a projection 54 formed on the inner surface of the outer tube hub 23. The outer tube hub 23 may be bonded to the outer tube 2 with an adhesive applied to the outer surface of the flection-preventing tube 50. For the material for forming the outer tube hub 23, there can be suitably used thermoplastic resin such as polycarbonate, polyamide, polysulfone, polyarylate and methacrylate-butylene-styrene copolymer.

The step of forming the opening 11 which is in communication with the lumen 6 of the outer tube 2, in the base end portion of the outer tube 2 may be taken place at any time after forming the outer tube 2. This step is preferably taken place after the step of attaching the base end portion of the expansible member 3 to the tip end portion of the outer tube 2 described later. The order of this step and the step of forming the inner tube 1 is optional.

Next, a step of forming the opening 9 which is in communication with the lumen 4 of the inner tube 1, in the base end portion of the inner tube 1 will be described. It is preferable to form the opening 9 by attaching the inner tube hub 22 having an opening to the base end portion of the inner tube 1. This case will be described with reference to Fig.6.

First, the flexion-preventing tube 60 is attached to one end portion of the inner tube 1. The flexion-preventing tube 60 consists of such a shrinkable tube that the inner diameter thereof after shrinkage is a little smaller than the outer diameter of the inner tube 1. The flexion-preventing tube 60 is attached by the manner that the shrinkable tube 60 is put on the one end portion of the inner tube 1 and then shrinked by heating, for example, with blowing hot air. In the embodiment shown in Fig.6, the rigidity imparting member 13 is disposed between the inner and outer tubes 1 and 2. One end portion of the rigidity imparting member 13 is fixed between the flexion-preventing tube 60 and the inner tube 1. As shown in Fig.6, the one end portion of the rigidity imparting member 13 can be fixed at the same time that the tube 60 is attached to the inner tube 1 by taking place the heat treatment described above after the one end portion of the rigidity imparting member 13 is located between the tube 60 and the inner tube 1.

The inner tube hub 22 is attached to the inner tube 1 to which the flexion-preventing tube 60 has been attached. This attachment of the inner tube hub 22 is taken place by using a lock member 62 which has an enlarged rear end portion. The outer diameter of the portion except the rear end portion of the lock member 62 is as large as the inner diameter of the inner tube 1. For attaching the inner tube hub 22 to the inner tube 1, after the lock member 62 is inserted in the rear end portion of the inner tube 1, the inner tube 1 is inserted from its tip end in the inner tube hub 22 till the enlarged rear end portion of the lock member 62 passes on a projection 64 formed on the inner surface of the inner tube hub 22. The inner tube hub 22 may be bonded to the inner tube 1 with an adhesive applied to the outer surface of the flexion-preventing tube 60. For the material for forming the inner tube hub, the same material as that of the outer tube hub can be suitably used. The step of forming the opening 9 which is in communication with the lumen 4 of the inner tube 1, in the base end portion of the inner tube 1 may be taken place at any time after forming the inner tube 1. The order of this step and the steps of forming the outer tube 2, forming the opening 11 which is in communication with the lumen 6 of the outer tube 2, in the base end portion of the outer tube 2, and forming the expansible member 3 is optional.

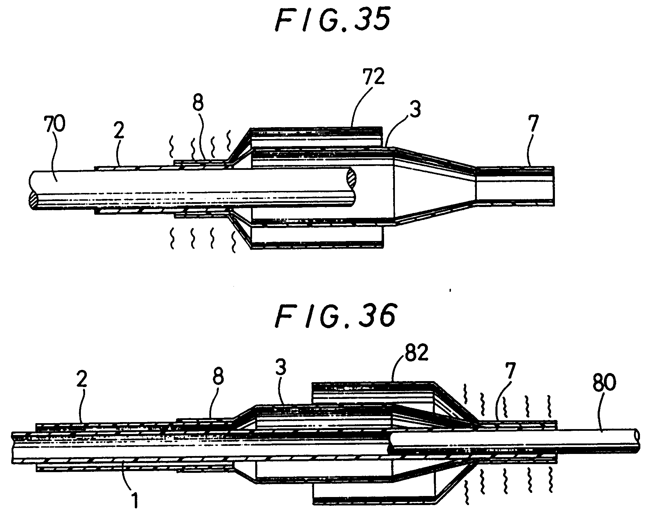

Next, the step of attaching the base end portion 8 of the expansible member 3 to the tip end portion of the outer tube 2 will be described. As shown in Fig.35, for attaching the base end portion 8 of the expansible member 3 to the tip end portion of the outer tube 2, a core mold 70 the outer diameter of which is as large as or a little smaller than the inner diameter of the outer tube 2 is inserted in the outer tube 2 from the tip or base end of the outer tube 2. Then, the expansible member 3 is put on the outer tube 2 from the tip end side of the core mold 70 such that the tip end of the base end portion 8 of the expansible member 3 corresponds to the tip end of the outer tube 2. A glass mold 72 for connecting is put on the base end portion 8 of the expansible member 3. The glass mold 72 is heated by a heater (not shown) to bond the base end portion 8 of the expansible member 3 to the tip end portion of the outer tube 2. In the case of using the expansible member 3 the base end portion 8 of which is shrinkable, it is easy to fix the base end portion 8 of the expansible member 3 to the tip end portion of the outer tube 2 because the base end portion 8 of the expansible member 3 shrinks by being heated through the glass mold 72 described above. After fixing the base end portion 8 of the expansible member 3 to the tip end portion of the outer tube 2, the glass mold 72 is left till it comes back to the room temperature. After this, the glass mold 72 is moved back from the connecting portion of the expansible member 3 and the outer tube 2, and the core mold 70 is removed. Although a glass mold is used in this embodiment, a metal mold for connecting may be used instead of the glass mold, for example. Also the core mold 70 may be made of metal. In that case, an electrode for generating a high frequency may be put on the base end portion 8 of the expansible member 3 to weld the base end portion 8 of the expansible member 3 to the tip end portion of the outer tube 2 by high frequency induction heating. The base end portion 8 of the expansible member 3 may be welded to the tip end portion of the outer tube 2 with supersonic wave. The step of attaching the base end portion 8 of the expansible member 3 to the tip end portion of the outer tube 2 may be taken place at any time after forming the outer tube 2 and the expansible member 3. The order of this step and the steps of forming the inner tube 1 and forming the opening 9 which is in communication with the lumen 4 of the inner tube 1, in the base end portion of the inner tube 1 Is optional. In the case of using a split mold, which is capable of being split along its axial direction, as the glass or metal mold as described above, further, in the case of welding with high frequency or supersonic wave, the order of this step and the step of forming the opening 11 which is in communication with the lumen 6 of the outer tube 2, in the base end portion of the outer tube 2 is optional. However, this step is preferably taken place after forming the opening 11 which is in communication with the lumen 6 of the outer tube 2, in the base end portion of the outer tube 2 because of decreasing the possibility of damaging the expansible member upon manufacturing.

Next, a step of connecting the inner tube hub 22 attached to the base end portion of the inner tube 1 and having the opening to the outer tube hub 23 attached to the base end portion of the outer tube 2 and having the opening will be described.

As shown in Fig.6, the inner tube 1 is inserted from its tip end in the outer tube hub 23 attached to the base end portion of the outer tube 2. The insertion of the inner tube 1 is performed from the rear end side of the outer tube hub 23. At this time, it is preferable that the inner tube 1 is inserted with a core member which is inserted in the inner tube 1 to prevent a flexion of the inner tube 1, and the tip end portion of the inner tube hub 22 is inserted into the rear end portion of the outer tube hub 23 and joined to the latter. It is also preferable that the inner and outer tube hubs 22 and 23 are surely bonded to each other with an adhesive applied to the connecting portion thereof. The step of connecting the inner tube hub 22 attached to the base end portion of the inner tube 1 and having the opening to the outer tube hub 23 attached to the base end portion of the outer tube 2 may be taken place at any time after the steps of forming the inner tube 1, disposing the inner tube hub 22 to the base end portion of the inner tube 1, forming the outer tube 2, and disposing the outer tube hub 23 to the base end portion of the outer tube 2. This step Is preferably taken place after forming the expansible member 3 and attaching the expansible member 3 to the outer tube 2.

Next, the step of attaching the tip end portion 7 of the expansible member 3 to the tip end portion of the inner tube 1 will be described.

Here, in case that the step of attaching the tip end portion 7 of the expansible member 3 to the tip end portion of the inner tube 1 is taken place after attaching the expansible member 3 to the outer tube 2 and connecting the inner tube hub 22 attached to the base end portion of the inner tube 1 and having the opening to the outer tube hub 23 attached to the base end portion of the outer tube 2 will be described.

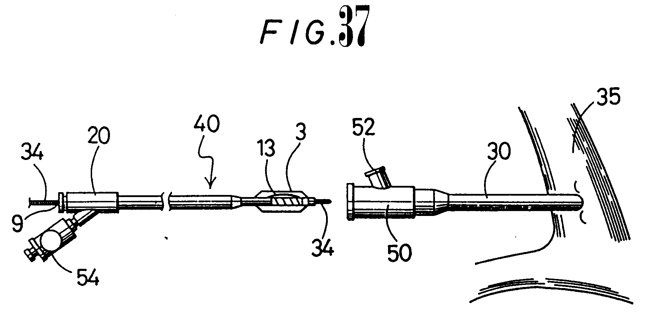

As shown in Fig.36, a core mold 80 the outer diameter of which is as large as or a little smaller than the inner diameter of the inner tube 1 is inserted in the inner tube 1 from the tip or base end of the inner tube 1. Because the expansible member 3 is attached to the outer tube 2, the inner tube 1 is inserted in the outer tube 2 and the inner and outer tube hubs 22 and 23 are connected to each other, the inner tube 1 protrudes beyond the tip of the outer tube 2 and the tip of the expansible member 3. Accordingly, the portion of the tip end portion of the inner tube 1 protruding beyond the tip of the expansible member 3 is cut off to fit in the tip of the expansible member 3. Then, a glass mold 82 for connecting is put from the tip end side of the core mold 80 on the tip end portion 7 of the expansible member 3. The glass mold 82 is heated by a heater (not shown) to bond the tip end portion 7 of the expansible member 3 to the tip end portion of the inner tube 1. In the case of using the expansible member 3 the tip end portion 7 of which is shrinkable, it is easy to fix the tip end portion 7 of the expansible member 3 to the tip end portion of the inner tube 1 because the tip end portion 7 of the expansible member 3 shrinks by being heated through the glass mold 82 described above. After fixing the tip end portion 7 of the expansible member 3 to the tip end portion of the inner tube 1, the glass mold 82 is left till it comes back to the room temperature. After this, the glass mold 82 is moved back from the connecting portion of the expansible member 3 and the inner tube 1, and the core mold 80 is removed. Although the glass mold is used in this embodiment, a metal mold for connecting may be used instead of the glass mold, for example. Also the core mold 80 may be made of metal. In that case, an electrode for generating a high frequency may be put on the tip end portion 7 of the expansible member 3 to weld the tip end portion 7 of the expansible member 3 to the tip end portion of the inner tube 1 by high frequency induction heating. The tip end portion 7 of the expansible member 3 may be welded to the tip end portion of the inner tube 1 with supersonic wave. The step of attaching the tip end portion 7 of the expansible member 3 to the tip end portion of the inner tube 1 is preferably taken place as the final step after attaching the expansible member 3 to the outer tube 2 and connecting the inner tube hub 22 attached to the base end portion of the inner tube 1 and having the opening to the outer tube hub 23 attached to the base end portion of the outer tube 2. In the case of using a split mold, which is capable of being split along its axial direction, as the glass or metal mold as described above, further, in the case of welding with high frequency or supersonic wave, this step may be taken place at any time after forming the inner tube 1 and the expansible member 3. The order of this step and the steps of forming the opening 9 which is in communication with the lumen 4 of the inner tube 1, in the base end portion of the inner tube 1, forming the outer tube 2, and forming the opening 11 which is in communication with the lumen 6 of the outer tube 2, in the base end portion of the outer tube 2 is optional.

Further, after attaching the tip end portion of the expansible member to the tip end portion of the inner tube as described above, the tip end portion of the inner tube is preferably processed so that the outer diameter of the tip end portion of the inner tube decreases in a tapering shape toward the tip end of the inner tube or the inner tube has a rounded tip end. Such a processing can be easily performed by the manner that the tip end portion of the inner tube is inserted in a mold (for example, a glass or metal mold) having an internal shape corresponding to the aimed tip end shape of the inner tube and the mold is heated to deform the tip end portion of the inner tube in accordance with the internal shape of the mold. The tip end portion of the inner tube may be processed by using a metal mold as the above-mentioned mold and applying a high frequency or supersonic wave to the mold.

Next, the operation of a catheter equipped with an expansible member according to the present invention will be described with reference to Figs. 37 through 41 using the catheter equipped with the expansible member of the embodiment shown in Figs.1 through 6.

Before administering a dilatating cure of a stricture portion which has occurred in a blood vessel, air in the catheter equipped with the expansible member is preferably removed as completely as possible. For this purpose, suction and injection means such as a syringe is attached to the second opening 11 of the catheter according to the present invention and liquid (X-ray contrast liquid, etc.) is charged in the syringe. By repeating suction and injection operations by syringe, the air in the second lumen and the expansible member is removed and replaced by the liquid.

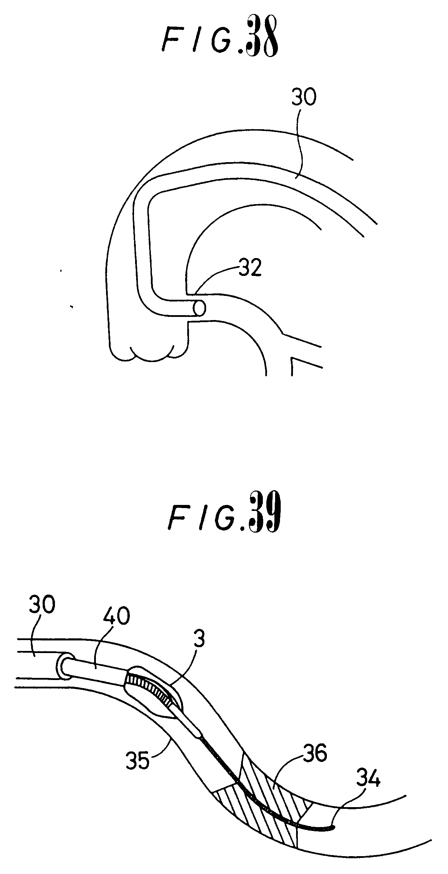

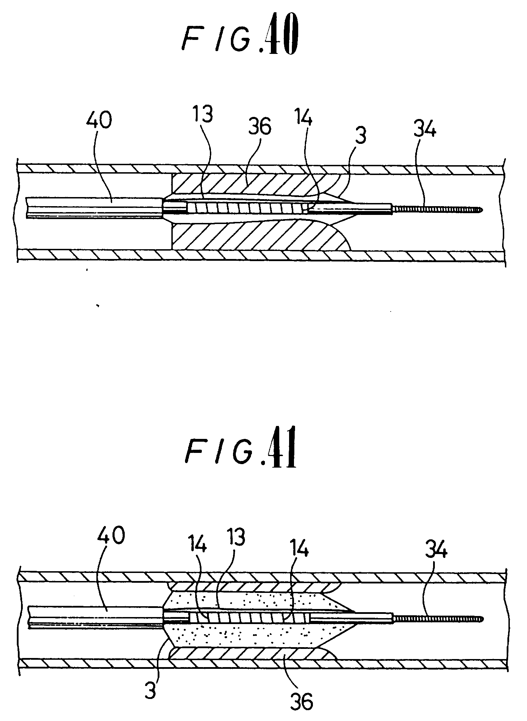

Upon inserting the catheter equipped with the expansible member into a blood vessel, at first, the blood vessel is insured by means of Seldinger method or the like, a guide wire for guide catheter (not shown) is then retained in the blood vessel, the guide catheter 30 is inserted into the blood vessel along it, as shown in Fig. 38 the guide catheter 30 is retained in the inlet 32 of the coronary artery including an aimed lesion part, and the guide wire for guide catheter is then withdrawn. As shown in Fig. 37,the catheter 40 equipped with the expansible member according to the present invention into which a guide wire 34 for catheter equipped with expansible member is inserted, is inserted through a Y-shaped connector 50 disposed at the rear end of the guide catheter 30. Insertion into the blood vessel is performed in a state that the guide wire 34 for catheter equipped with expansible member is protruded beyond the tip of the catheter 40 equipped with the expansible member by several centimeters. The catheter 40 equipped with the expansible member advances in the guide catheter 30 and enters the blood vessel 35 including the aimed lesion part as shown in Fig.39. The guide wire 34 for catheter equipped with expansible member is then advanced to the aimed lesion part, passed through the stricture portion 36 and retained. The catheter 40 equipped with the expansible member advances in the blood vessel 35 along the guide wire 34 for catheter equipped with expansible member. When the catheter 40 equipped with the expansible member reachs the stricture portion 36, the expansible member 3 is positioned in the stricture portion 36 under X-ray perspection by using the X-ray impermeable marker 14 disposed on the inner tube 1 as a reference mark as shown in Fig.40. Subsequently, vasographic contrast liquid is injected at a pressure of several atmospheres for example ten or more atmospheres by means of an injector 54 equipped with a pressure gauge connected to the second opening forming the injection port of the catheter 40 equipped with the expansible member to compress and expand the stricture portion 36 as shown in Fig.41. The contrast liquid is injected through a contrast liquid injection port 52 of the Y-shaped connector 50 of the guide catheter 30 to confirm the state of blood stream on the peripheral side by the X-ray perspection. When an improvement of the blood flow on the peripheral side is recognized, the catheter 40 equipped with the expansible member and the guide wire 34 for catheter equipped with expansible member are withdrawn and then the guide catheter is withdrawn and blood is stopped under pressure to complete the operation.

As described above, a catheter equipped with an expansible member according to the present invention comprises an inner tube having a base end portion and a first lumen whose tip is open; an outer tube capable of inserting said inner tube therein, having a base end portion and the tip thereof at a position recessed by a predetermined distance from the tip of said inner tube, and forming a second lumen between it and the outer surface of said inner tube; a foldable expansible member having a tip end portion and a base end portion, said tip end portion of said expansible member being attached to said inner tube, said base end portion of said expansible member being attached to said outer tube, said expansible member communicating with said second lumen at a portion near said base end portion of said expansible member; a first opening formed in said base end portion of said inner tube to communicate with said first lumen; a second opening formed in said base end portion of said outer tube to communicate with said second lumen; and a rigidity imparting member consisting of a linear member extending in the axial direction disposed in said second lumen. Therefore, particularly because the rigidity imparting member is disposed in the second lumen, an extreme flexion and meandering of the catheter is prevented without decrease of the flexibility of the catheter, especially the elastic deformability in the lateral direction with respect to the axial direction of the catheter. Therefore, the force applied to the base end portion of the catheter is not absorbed at the meandering portion. Thus, the force applied to the base end portion of the catheter for advancing the tip end portion thereof to a stricture portion inside a blood vessel is surely transmitted to the tip end portion so the operability of the catheter is good. In addition, because the second lumen which is in communication with the portion near the base end portion of the expansible member and into which expansion fluid for the expansible member is injected is formed between the inner and outer tubes and has a relatively large volume, even in the case that the expansion fluid has a high flow resistance such as vasographic contrast liquid, it can be easily injected.

Further, a catheter equipped with an expansible member according to the present invention comprises a catheter tube having a tip end portion, a base end portion, a first lumen at least the tip of which is open, a second lumen open at a position recessed by a predetermined distance from said tip of said first lumen; a foldable expansible member having a tip end portion and a base end portion, said tip end portion of said expansible member being attached to said tip end portion of said catheter tube, said base end portion of said expansible member being attached to a portion near an opening portion formed near the tip of said catheter tube, said expansible member communicating with said second lumen; a first opening formed in said base end portion of said catheter tube to communicate with said first lumen; a second opening formed in said base end portion of said catheter tube to communicate with said second lumen; and a rigidity imparting member consisting of a linear member extending in the axial direction disposed in at least one of said first and second lumens. Therefore, like the catheter described before, because the rigidity imparting member is disposed in the second lumen, an extreme flexion and meandering of the catheter is prevented without decrease of the flexibility of the catheter, especially the elastic deformability in the lateral direction with respect to the axial direction of the catheter. Therefore, the force applied to the base end portion of the catheter is not absorbed at the meandering portion. Thus, the force applied to the base end portion of the catheter for advancing the tip end portion thereof to a stricture portion inside a blood vessel is surely transmitted to the tip end portion so the operability of the catheter is good.

## Claims

1. A catheter equipped with an expansible member comprising an inner tube (1) having a base end portion and a first lumen (4) whose tip is open; an outer tube (2) receiving said inner tube therein, having a base end portion and the tip thereof at a position recessed by a predetermined distance from the tip of said inner tube (1), and forming a second lumen (6) between it and the outer surface of said inner tube (1); a foldable expansible member (3) having a tip end portion (7) and a base end portion (8), said tip end portion (7) of said expansible member being attached to said inner tube (1), said base end portion (8) of said expansible member being attached to said outer tube (2), said expansible member (3) communicating with said second lumen (6) at a portion near said base end portion of said expansible member; a first opening (9) formed in said base end portion or said inner tube (1) to communicate with said first lumen (4); a second opening (11) formed in said base end portion of said outer tube (2) to communicate with said second lumen (6); and a rigidity imparting member (13) extending in the axial direction characterized in that said rigidity imparting member (13) consists of a linear member disposed in said second lumen (6), the tip end portion of the rigidity imparting member (13) being fixed to the tip end portion of the inner tube (1), the base end portion of the rigidity imparting member (13) being fixed to the inner or outer tube and the intermediate portion between the tip end and the base end portions of said rigidity imparting member (13) being fixed neither to the inner nor to the outer tube and being of linear shape.

2. A catheter equipped with an expansible member as set forth in claim 1, wherein the rigidity of the base end portion of said rigidity imparting member (13) is higher than that of the tip end portion of said rigidity imparting member.

3. A catheter equipped with an expansible member as set forth in claim 1, wherein the cross section of the base end portion of said rigidity imparting member (13) is larger than that of the tip end portion of said rigidity imparting member.

4. A catheter equipped with an expansible member as set forth in any preceding claim, wherein said rigidity importing member (13) is a strand wire consisting of several fine metal wires twisted together.

5. A catheter equipped with an expansible member as set forth in any preceding claim, wherein the tip end portion of said rigidity imparting member (13) is fixed by winding it around the outer surface of the inner tube (1).

6. A catheter equipped with an expansible member comprising a catheter tube (10) having a tip end portion, a base end portion, a first lumen (4) the tip or which is open, a second lumen (6) open at a position recessed by a predetermined distance from said tip of said first lumen; a foldable expansible member (8) having a tip end portion (7) and a base end portion (8), said tip end portion of said expansible member being attached to said tip end portion of said catheter tube, said base end portion of said expansible member being attached to a portion near an opening portion (12) formed near the tip of said catheter tube, said expansible member communicating with said second lumen, a first opening (9) formed in said base end portion of said catheter tube to communicate with said first lumen (4); a second opening (11) formed in said base end portion of said catheter tube to communicate with said second lumen (6); and a rigidity imparting member (13) extending in the axial direction, characterized in that said rigidity imparting member (13) consists of a linear member disposed in at least one of said first and second lumens, and the tip end portion of the rigidity imparting member (13) is fixed to the tip end portion of said catheter tube (10), the base end portion of the rigidity imparting member is fixed to the base end portion of said catheter tube (10), and the intermediate portion between the tip end and the base end portions of said rigidity imparting member (13) in not fixed to the catheter tube and is of linear shape.

7. A catheter equipped with an expansible member as set forth in claim 6, wherein the rigidity of the base end portion of said rigidity imparting member (13) is higher than that of the tip end portion of said rigidity imparting member.

8. A catheter equipped with an expansible member as set forth in claim 6, wherein the cross section of the base end portion of said rigidity imparting member (19) is larger than that of the tip end.

9. A catheter equipped with an expansible member as set forth in anyone of claims 6 to 8, wherein said rigidity imparting member is a strand wire consisting of several fine metal wires twisted together.

## Patentansprüche

1. Katheter, mit einem ausdehnbaren Element, umfassend ein inneres Rohr (1) mit einem proximalen Abschnitt und einem ersten Hohlraum (4), dessen distales Ende offen ist; ein das innere Rohr darin aufnehmendes äußeres Rohr (2), das einen proximalen Abschnitt aufweist und dessen distales Ende um eine vorgegebene Strecke von dem distalen Ende des inneren Rohres (1) abgesetzt ist, und das zwischen ihm und der Außenfläche des inneren Rohres (1) einen zweiten Hohlraum (6) bildet; ein faltbares, ausdehnbares Element (3) mit einem distalen Abschnitt (7) und einem proximalen Abschnitt (8), wobei der distale Abschnitt (7) des ausdehnbaren Elements an dem inneren Rohr (1) befestigt ist, der proximale Abschnitt (8) des ausdehnbaren Elementes an dem äußeren Rohr (2) befestigt ist, das ausdehnbare Element (3) in einem Bereich in der Nähe des proximalen Abschnittes des ausdehnbaren Elementes mit dem zweiten Hohlraum (6) in Verbindung steht; eine erste Öffnung (9), die in dem proximalen Abschnitt des inneren Rohres (1) gebildet ist, um mit dem ersten Hohlraum (4) in Verbindung zu stehen; eine zweite Öffnung (11), die in dem proximalen Abschnitt des äußeren Rohres (2) zur Verbindung mit dem zweiten Hohlraum (6) gebildet ist; und ein Versteifungselement (13), das sich in axialer Richtung erstreckt
**dadurch gekennzeichnet**, daß
das Versteifungselement (13) aus einem geradlinigen Element besteht, das in dem zweiten Hohlraum (6) angeordnet ist, wobei der distale Abschnitt des Versteifungselements (13) an dem distalen Abschnitt des inneren Rohres (1) befestigt ist, der proximale Abschnitt des Versteifungselements (13) an dem inneren oder äußeren Rohr befestigt ist und der zwischen dem distalen Abschnitt und dem proximalen Abschnitt des Versteifungselements (13) liegende Zwischenabschnitt weder an dem inneren noch an dem äußeren Rohr befestigt ist und eine geradlinige Form aufweist.

2. Katheter mit einem ausdehnbaren Element nach Anspruch 1, **dadurch gekennzeichnet, daß** die Steifigkeit des proximalen Abschnittes des Versteifungselements (13) größer ist als die des distalen Abschnittes des Versteifungselements.

3. Katheter mit einem ausdehnbaren Element nach Anspruch 1, **dadurch gekennzeichnet, daß** der Querschnitt des proximalen Abschnittes des Versteifungselements (13) größer ist als der des distalen Abschnittes des Versteifungselements.

4. Katheter mit einem ausdehnbaren Element nach einem der vorhergehenden Ansprüche, **dadurch gekennzeichnet, daß** das Versteifungselement (13) eine Litze ist, die aus mehreren dünnen, miteinander verdrillten Metalldrähten besteht.

5. Katheter mit einem ausdehnbaren Element nach einem der vorhergehenden Ansprüche , **dadurch gekennzeichnet, daß** der distale Abschnitt des Versteifungselements (13) befestigt ist, indem er um die Außenfläche des inneren Rohres (1) gewickelt ist.

6. Katheter mit einem ausdehnbaren Element, umfassend ein Katheterrohr (10) mit einem distalen Abschnitt, einem proximalen Abschnitt, einem ersten Hohlraum (4), dessen distales Ende offen ist, einem zweiten Hohlraum (6), der an einer um eine vorgegebene Strecke von dem distalen Ende des ersten Hohlraumes abgesetzten Position offen ist; ein faltbares, ausdehnbares Element (3) mit einem distalen Abschnitt (7) und einem proximalen Abschnitt (8), wobei der distale Abschnitt des ausdehnbaren Elements an dem distalen Abschnitt des Katheterrohres befestigt ist, der proximale Abschnitt des ausdehnbaren Elements mit einem Abschnitt nahe einem Öffnungsabschnitt (12) befestigt ist, der Nähe des distalen Endes des Katheterrohres gebildet ist, wobei das ausdehnbare Element mit dem zweiten Hohlraum in Verbindung steht; eine erste Öffnung (9), die in dem proximalen Abschnitt des Katheterrohres zur Verbindung mit dem ersten Hohlraum (4) gebildet ist; eine zweite Öffnung (11), die in dem proximalen Abschnitt des Katheterrohres zur Verbindung mit dem zweiten Hohlraum (6) gebildet ist; und ein Versteifungselement (13), das sich in axialer Richtung erstreckt, **dadurch gekennzeichnet, daß** das Versteifungselement (13) aus einem geradlinigen Element besteht, das wenigstens im ersten oder zweiten Hohlraum angeordnet ist, daß der distale Abschnitt des Versteifungselements (13) an dem distalen Abschnitt des Katheterrohrs (10) befestigt ist, der proximale Abschnitt des Versteifungselements an dem proximalen Abschnitt des Katheterrohres (10) befestigt ist, und daß der zwischen dem distalen Abschnitt und dem proximalen Abschnitt des Versteifungselements (13) liegende Zwischenabschnitt nicht an dem Katheterrohr befestigt ist und eine geradlinige Form aufweist.

7. Katheter mit einem ausdehnbaren Element nach Anspruch 6, **dadurch gekennzeichnet, daß** die Steifigkeit des proximalen Abschnittes des Versteifungselements (13) größer ist als die des distalen Abschnittes des Versteifungselements.

8. Katheter mit einem ausdehnbaren Element nach Anspruch 6, **dadurch gekennzeichnet, daß** der Querschnitt des proximalen Abschnittes des Versteifungselements (13) größer ist als der des distalen Endes.

9. Katheter mit einem ausdehnbaren Element nach einem der Ansprüche 6 bis 8, **dadurch gekennzeichnet, daß** das Versteifungselement eine Litze ist, die aus mehreren dünnen, miteinander verdrillten Metalldrähten besteht.

## Revendications

1. Cathéter muni d'un élément expansible comprenant un tube intérieur (1) comportant une partie d'extrémité proximale et une première lumière (4) dont l'extrémité distale est ouverte; un tube extérieur (2) logeant ledit tube intérieur, comportant une partie d'extrémité proximale et dont l'extrémité distale se trouve à un endroit en retrait d'une distance prédéterminée de l'extrémité distale dudit tube intérieur (1), et formant une deuxième lumière (6) entre lui-même et la surface extérieure dudit tube intérieur (1); un élément expansible pliable (3) comportant une partie d'extrémité distale (7) et une partie d'extrémité proximale (8), ladite partie d'extrémité distale (7) dudit élément expansible étant fixée audit tube intérieur (1), ladite partie d'extrémité proximale (8) dudit élément expansible étant fixée audit tube extérieur (2), ledit élément expansible (3) communiquant avec ladite deuxième lumière (6) à une partie située près de ladite partie d'extrémité proximale dudit élément expansible; une première ouverture (9) formée dans ladite partie d'extrémité proximale dudit tube intérieur (1) pour communiquer avec ladite première lumière (4); une deuxième ouverture (11) formée dans ladite partie d'extrémité proximale dudit tube extérieur (2) pour communiquer avec ladite deuxième lumière (6); et un élément (13) conférant de la rigidité et s'étendant dans la direction axiale, caractérisé en ce que ledit élément (13) conférant de la rigidité consiste en un élément linéaire disposé dans ladite deuxième lumière (6), la partie d'extrémité distale de l'élément (13) conférant de la rigidité étant fixée à la partie d'extrémité distale du tube intérieur (1), la partie d'extrémité proximale de l'élément (13) qui confère de la rigidité étant fixée au tube intérieur ou au tube extérieur et la partie intermédiaire entre les parties d'extrémité distale et d'extrémité proximale dudit élément (13)qui confère de la rigidité n'étant fixée ni au tube intérieur ni au tube extérieur et ayant une forme linéaire.

2. Cathéter muni d'un élément expansible selon la revendication 1, dans lequel la rigidité de la partie d'extrémité proximale dudit élément (13) conférant de la rigidité est plus grande que celle de la partie d'extrémité distale dudit élément conférant de la rigidité.

3. Cathéter muni d'un élément expansible selon la revendication 1, dans lequel la section transversale de la partie d'extrémité proximale dudit élément (13) communiquant de la rigidité est plus grande que celle de la partie d'extrémité distale dudit élément conférant de la rigidité.

4. Cathéter muni d'un élément expansible selon l'une quelconque des revendications précédentes, dans lequel ledit élément (13) conférant de la rigidité et un fil métallique toronné consistant en plusieurs fils métalliques fins tordus ensemble.

5. Cathéter muni d'un élément expansible selon l'une quelconque des revendications précédentes, dans lequel la partie d'extrémité distale dudit élément (13) conférant de la rigidité est immobilisée par le fait qu'elle est enroulée autour de la surface extérieure du tube intérieur (1).

6. Cathéter muni d'un élément expansible comprenant un tube (10) de cathéter comportant une partie d'extrémité distale, une partie d'extrémité proximale, une première lumière (1) dont l'extrémité distale est ouverte, une deuxième lumière (6) ouverte à un endroit en retrait d'une distance prédéterminée de ladite extrémité distale de ladite première lumière; un élément expansible pliable (8) comportant une partie d'extrémité distale (7) et une partie d'extrémité proximale (8), ladite partie d'extrémité distale dudit élément expansible étant fixée à ladite partie d'extrémité distale dudit tube de cathéter, ladite partie d'extrémité de base dudit élément expansible étant fixée à une partie située près d'une partie ouverture (12) formée près de l'extrémité distale dudit tube de cathéter, ledit élément expansible communiquant avec ladite deuxième lumière, une première ouverture (9) formée dans ladite partie d'extrémité proximale dudit tube de cathéter pour communiquer avec ladite première lumière (4); une deuxième ouverture (11) formée dans ladite partie d'extrémité proximale dudit tube de cathéter pour communiquer avec ladite deuxième lumière (6); et un élément (13) conférant de la rigidité s'étendant dans la direction axiale, caractérisé en ce que ledit élément (13) conférant de la rigidité consiste en un élément linéaire disposé dans au moins une desdites première et deuxième lumières, et la partie d'extrémité distale de l'élément (13) conférant de la rigidité est fixée à la partie d'extrémité distale dudit tube (10) de cathéter, la partie d'extrémité proximale de l'élément conférant de la rigidité est fixée à la partie d'extrémité proximale dudit tube (10) de cathéter, et la partie intermédiaire entre la partie d'extrémité distale et la partie d'extrémité proximale dudit élément (13) conférant de la rigidité n'est pas fixé au tube de cathéter et a une forme linéaire.

7. Cathéter muni d'un élément expansible selon la revendication 6, dans lequel la rigidité de la partie d'extrémité proximale dudit élément (13) conférant de la rigidité est plus grande que celle de la partie d'extrémité distale dudit élément conférant de la rigidité.

8. Cathéter muni d'un élément expansible selon la revendication 6, dans lequel la section transversale de la partie d'extrémité proximale dudit élément (19) conférant de la rigidité est plus grande que celle de partie d'extrémité distale.

9. Cathéter muni d'un élément expansible selon l'une quelconque des revendications 6 à 8, dans lequel ledit élément conférant de la rigidité est un fil métallique toronné consistant en plusieurs fils métalliques fins tordus ensemble.
